Europäisches Patentamt

European Patent Office

Office européen des brevets

(11) Publication number: **0 472 220 A1**

## EUROPEAN PATENT APPLICATION

(21) Application number: **91114248.7**

(22) Date of filing: **23.08.91**

(51) Int. Cl.⁵: **C07K 7/18**, A61K 37/42, C07K 1/04, C07K 1/06

(30) Priority: **24.08.90 US 572722**

(43) Date of publication of application: **26.02.92 Bulletin 92/09**

(84) Designated Contracting States: **AT BE CH DE DK ES FR GB GR IT LI LU NL SE**

(71) Applicant: **SYNTEX (U.S.A.) INC. 3401 Hillview Avenue P.O. Box 10850 Palo Alto California 94303(US)**

(72) Inventor: **Nestor, John Joseph, Jr. 20937 Fairwoods Court Cupertino, CA 95014(US)**
Inventor: **Ernest, Michael Jeffrey 210 Sleeper Avenue Mountain View, CA 94040(US)**
Inventor: **Ho, Teresa Hing 495 S. Clark Avenue Los Altos, CA 94024(US)**

(74) Representative: **Barz, Peter, Dr. et al Patentanwälte Dipl.-Ing. G. Dannenberg Dr. P. Weinhold, Dr. D. Gudel Dipl.-Ing. S. Schubert, Dr. P. Barz Siegfriedstrasse 8 W-8000 München 40(DE)**

(54) Bradykinin antagonists.

(57) Analogs of bradykinin comprising derivatives of arginine or homoarginine residues exhibit bradykinin antagonist activity.

This invention relates to compounds which act as antagonists of the biological activities of bradykinin. In particular, the invention relates to analogs of bradykinin containing derivatized arginine or homoarginine N-terminal residues.

Bradykinin is a naturally occurring nonapeptide. Bradykinin and its related substances, Lys-bradykinin (kallidin) and Met-Lys-bradykinin, are enzymatically cleaved from precursor molecules (e.g. kallikrein) found in plasma or in tissues (see Burch et al., **Med. Res. Rev.** 1990, **10**: 237) in response to tissue injury, trauma, or other signals. Bradykinin exerts a number of effects which lead to pain and inflammation in tissues, either directly by interaction with specific receptors or indirectly by activation of the arachidonic acid cascade to produce proinflammatory prostaglandins, leukotrienes, and platelet activating factor (PAF). In addition, bradykinin can contract intestinal and bronchial smooth muscle to produce diarrhea and asthma. Bradykinin involvement in the symptoms of rhinitis (Proud et al., **J. Clin. Invest.** 1983, **72**: 1678), shock (Weipert et al., **Br. J. Pharmacol.** 1988, **94**: 282), inflammation-induced bone resorption (Lerner et al., **Arthritis and Rheumatism** 1987, **30**: 530), angina pectoris (Steranka, **Proc. Natl. Acad. Sci. (USA)** 1988, **85**: 3245), and other disease processes, such as pancreatitis, carcinoid syndrome, clotting, and complement-mediated reactions, is well known to those skilled in the art.

Considerable evidence exists for the role of bradykinin as a physiological indicator of pain. Bradykinin is well-known as a highly potent algesic agent (Collier et al., **Br. J. Pharmacol.** 1963, 21:151). In humans, bradykinin causes a burning, stinging pain after application to a blister base as well as after intradermal, intra-arterial, or interperitoneal injection (W. G. Clark in Bradykinin, Kallidin and Kallikrein, Vol. XXV, Supplement, Handbook of Experimental Pharmacology, E. G. Erdos, Ed., Springer-Verlag, New York, 1979, pp. 569-607). In addition, bradykinin is present in damaged tissues at concentrations sufficient to induce pain (Kellermeyer et al., **N. Engl. J. Med.** 1968, 279:859).

Bradykinin, when injected into the skin of animals and humans, elicits responses that mimic the four major signs of inflammation (redness, swelling, heat and pain) as well as accumulation of leukocytes (Marceau et al., **Gen. Pharmacol.,** 1983, 14:209).

The production of bradykinin and associated pain in angina has been reported (Kimura et al., **Am. Heart J.**, **1973**, 85:635 and Staszewska-Barczak et al., **Cardiovascular Res.**, **1976**, 10:314). Bradykinin and prostaglandins acting in concert may provide the natural stimulus for excitation of the sensory receptors signalling the pain of myocardial ischemia.

Kinins are reported to be generated in nasal secretions during allergic reactions and during induced rhinovirus colds. Administration of bradykinin to the nasal mucosa induces symptoms of rhinitis and a sore throat. (Proud et al., **Am. Rev. Respir. Dis.** 1988, 137:613).

Lerner et al., have reported that bradykinin at and above 3 nM causes a dose-dependent stimulation of bone mineral mobilization and matrix degradation. These findings suggest that generation of bradykinin in inflammatory lesions of rheumatoid arthritis and periodontitis may contribute to the bone resorptive process seen in the joints and alveolar bone (Lerner et al**., Arthritis and Rheumatism, 1987**, 30:530).

Circulating levels of immunoreactive bradykinin are reported to be elevated in clinical models of acute (oral surgery) and chronic (rheumatoid arthritis) inflammation. Patients with rheumatoid arthritis have circulating levels of bradykinin approximately two to three times higher than those observed in control patients (Hargreaves et al., **Clin. Pharmacol. Ther.** 1988, 44:613-621).

Stewart and Vavrek in U. S. Patent No. 4,693,993, issued 9/15/87, and in U. S. Patent No. 4,801,613, issued 1/31/89, describe the role of bradykinin in pathological conditions such as septic shock, acute pancreatitis, hereditary angioneurotic edema, post-gastrectomy dumping syndrome, carcinoid syndrome, anaphylactic shock, reduced sperm motility, and other conditions. Bradykinin and bradykinin related kinins may also be injected as a result of stings and bites. The Stewart et al. patents disclose modified bradykinins which behave as bradykinin antagonists. The critical modification involved the replacement of the L-proline at position 7 with aromatic amino acids of D-configuration.

Workers have sought for many years to design potent, long-acting pure antagonists of bradykinin (Stewart, Handbook of Experimental Pharmacology, Vol. 25 (Suppl.), Springer Verlag, p. 227, 1979). Vavrek et al. (**Peptides 1985**, **6**: 161) have shown that analogs in which the proline residue at position 7 of the bradykinin molecule is replaced by a D-phenylalanine residue are bradykinin antagonists with moderate potency. Such antagonists still suffer from two fundamental problems: rapid cleavage by proteolysis at several positions, including the 8-9 bond, and mast cell degranulation (P. Devillier et al., **Eur. J. Pharmacol. 1988*, 149*: 137).

Gardner et al. in European Patent Publication No. 0 334 244, published 9/27/89, report that the peptides of the Stewart et al. '993 patent exhibit bradykinin agonist or mixed agonist/antagonist activity when assayed by certain analgesic and antiinflammatory in vivo procedures. Gardner et al. suggest bradykinin analogs in which either position 2, position 3, or both consist of an L-acidic, L-amide, or L-hydroxamate amino acid residue; position 7 consists of a D-aromatic amino acid residue; and position 4 preferably consists of an L-aliphatic or D-cyclic amino acid residue.

The half-life of bradykinin in the systemic circulation is less than 30 seconds (Ferreira et al., **Brit. J. Pharmacol. Chemotherap. 1967,** 30:417). The addition of the dipeptide Lys-Lys to the N-terminal of bradykinin agonists improves resistance to in vivo destruction on passage through the pulmonary circulation (Roblero et al., **Res. Comm. Pathol. 1973,** 6:207).

Stewart et al. in International Patent Application Nos. PCT/US88/02959 and PCT/US88/02960, both published March 9, 1989 as International Patent Publication Nos. WO 89/01780 and WO 89/01781, respectively, report that the replacement of arginine at the 1 and 9 positions, along with certain C-terminal and N-terminal extensions, increases enzyme resistance, antagonist potency and specificity.

In addition, Breipohl et al. have reported potent bradykinin antagonists with structures having substituents such as D-Tic in position 7 and Pro or Oic in position 8 (Peptidergic Receptors and Peptide Processing as Therapeutic Targets, Nice, France, April 8-11, 1990). The optimal compound reported was D-Arg-Arg-Pro-Hyp-Gly-Thi-Ser-Tic-Oic-Arg, designated as Hoe 140.

The disclosures of these, and all other documents cited in the specification of this application, are incorporated herein by reference.

It is an object of this invention to provide bradykinin antagonists of high potency and good resistance to proteolysis, while being less prone to induce histamine release by mast cell degranulation.

This invention comprises compounds of the general formula:

A-(B)$_m$-(C)$_n$-T-E-E-C-F-G-I-J-K    (I)

wherein:

A is H, acyl or glycosyl;

B is D-Arg, Arg(R$_1$), D-Arg(R$_1$), hArg(R$_1$), D-hArg(R$_1$), Arg(R$_1$,R$_2$), D-Arg(R$_1$,R$_2$), hArg(R$_1$,R$_2$), or D-hArg-(R$_1$,R$_2$), where R$_1$ is alkyl or fluoroalkyl and R$_2$ is cyano, alkyl, or fluoroalkyl;

C is $\beta$-Ala, Gly, or aza-Gly;

T is Arg or B;

E is Hyp or Pro;

F is Nal(1), Nal(2), Phe, Phe(Cl), Phe(F$_5$), Thi, Trp, or Tyr(OMe);

G is Gly, D-Phe, Ser, or D-Thi;

I is D-Ala, D-Dic, D-Hyp, D-Nal(1), D-Nal(2), D-Ohc, D-Oic, D-Pal(3), D-Phe, D-Phe(Cl), D-Pip, D-Pro, D-Thi, D-Thp, D-Tic, D-Trp, D-Tyr, D-Tyr(Me), D-$\alpha$MeNal(2), D-$\alpha$MePhe, or D-$\alpha$MePhe(Cl);

J is Dic, Hyp, Nal(1), Nal(2), Ohc, Oic, Phe, Phe(F$_5$), Phe(Cl), Pip, Pro, Thi, Thp, Tic, Tyr(Me), Tyr(Et), $\alpha$MeNal(2), $\alpha$MePhe, or $\alpha$MePhe(Cl);

K is Arg or B;

m is 1, 2, 3, 4, or 5; and

n is 0, 1, or 2,

and the pharmaceutically acceptable salts thereof.

This invention also encompasses methods for preparing the compounds of the invention, including the pharmaceutically acceptable salts. The methods comprise removing protecting groups and, if present, covalently bound solid supports, from a protected polypeptide to afford the compounds, optionally followed by ion exchange and chromatography to afford their pharmaceutically acceptable salts.

Further this invention comprises compositions for treatment of an individual wherein the pharmaceutical composition comprises an effective amount of a compound of the invention and a compatible pharmaceutical carrier.

A key feature of the compounds of this invention is the incorporation of alkylated and fluoroalkylated analogs of arginine into the bradykinin structure, thereby blocking enzymatic degradation, diminishing the induction of histamine release by mast cell degranulation, and further increasing the duration of action of the compounds by causing them to depot in the body. These advantages are manifested by improved performance in recognized assays, particularly in vivo assays, in which compounds of this invention exhibit high potency and good stability.

### Abbreviations and Definitions

For convenience in describing and claiming this invention, conventional abbreviations for the various common amino acids (as generally accepted in the peptide art and as recommended by the IUPAC-IUB Commission on Biochemical Nomenclature, **Biochem. J. 1984, 219**: 345) are used. All peptide sequences disclosed and/or claimed herein are written according to the generally accepted convention whereby the *N*-terminal amino acid is on the left and the *C*-terminal amino acid is on the right.

The abbreviations for the chiral amino acids herein represent L-amino acids unless the acids are designated as D- or D,L-. Certain amino acids, both natural (e.g. glycine) and non-natural, are achiral.

Substituted amino acids are designated as the appropriate parent amino acid with the substituent in parentheses, or by three-letter codes. For example:

$Arg(R_1)$ and $hArg(R_1)$ represent arginine and homoarginine, respectively, with an $R_1$ substituent on the $\omega$-nitrogen of the guanidino moiety; and

$Arg(R_1,R_2)$ and $hArg(R_1,R_2)$ represent arginine and homoarginine, respectively, with an $R_1$ substituent on the $\omega$-nitrogen and an $R_2$ substituent on the $\omega'$-nitrogen of the guanidino moiety.

Specific abbreviations of non-naturally occurring amino acids are useful in describing the invention. Representative non-naturally occuring amino acids include the following:

| Amino acid residue | Abbreviation |
|---|---|
| decahydroisoquinoline-<br>3-carboxylic acid | Dic |
| hydrazinecarboxylic acid (azaglycine) | aza-Gly |
| 4-hydroxyproline | Hyp |
| 3-(1-naphthyl)alanyl | Nal(1) |
| 3-(2-naphthyl)alanyl | Nal(2) |
| octahydrocyclopenta[b]pyrrole-<br>2-carboxylic acid | Ohc |
| octahydroindole-2-carboxylic acid | Oic |
| 3-(p-chlorophenyl)alanyl | Phe(Cl) |
| 3-(p-fluorophenyl)alanyl | Phe(F) |
| 3-(pentafluorophenyl)alanyl | $Phe(F_5)$ |
| piperidine-2-carboxylic acid<br>(2-pipecolic acid) | Pip |
| 3-(2-thienyl)alanine | Thi |
| tetrahydrothiazole-4-carboxylic acid<br>(4-thiaproline) | Thp |
| 1,2,3,4-tetrahydroisoquinoline-<br>3-carboxylic acid | Tic |
| O-methyltyrosine | Tyr(OMe) |
| $N^G$-ethylarginyl | Mea, Arg(Et) |
| $N^G$-ethylhomoarginyl | Meh, hArg(Et) |
| $N^G$-propylhomoarginyl | Prh, hArg(Pr) |
| $N^G$-isopropylhomoarginyl | Iph, hArg($i$Pr) |
| $N^G$-butylhomoarginyl | Mbh, hArg(Bu) |
| $N^G$-heptylhomoarginyl | Hha, hArg(heptyl) |
| $N^G,N^{G'}$-dimethylarginyl | Dma, $Arg(Me_2)$ |
| $N^G,N^{G'}$-dimethylhomoarginyl | Dmh, $hArg(Me_2)$ |
| $N^G,N^{G'}$-diethylhomoarginyl | Deh, $hArg(Et_2)$ |
| $N^G,N^{G'}$-dipropylhomoarginyl | Dph, $hArg(Pr_2)$ |
| $N^G,N^{G'}$-diisopropylhomoarginyl | Dih, $Arg(iPr_2)$ |

| | |
|---|---|
| $N^G,N^{G'}$-dihexylhomoarginyl | Dhh, hArg(hexyl$_2$) |
| $N^G,N^{G'}$-dicyclohexylhomoarginyl | Dch, hArg(cyclohexyl$_2$) |
| $N^G,N^{G'}$-ethanohomoarginyl | Eha, hArg(CH$_2$)$_2$ |
| $N^G,N^{G'}$-propanohomoarginyl | Pha, hArg(CH$_2$)$_3$ |
| $N^G,N^{G'}$-bis-(2,2,2-trifluoroethyl)-homoarginyl | Bth, hArg(CH$_2$CF$_3$)$_2$ |
| $N^G$-hexyl-$N^{G'}$-methylhomoarginyl | Hmh, hArg(hexyl,methyl) |
| $N^G$-butyl-$N^{G'}$-methylhomoarginyl | Bmh, hArg(butyl,methyl) |
| $N^G$-butyl-$N^{G'}$-cyanohomoarginyl | hArg(Bu,CN) |
| $N^G$-butyl-$N^{G'}$-cyanomathylhomoarginyl | hArg(Bu,CH$_2$CN) |
| $N^G,N^{G'}$-diisopropylarginyl | Dia, Arg($i$Pr$_2$) |
| $N^G,N^{G'}$-dicyclohexylhomoarginyl | Dca, Arg(cyclohexyl$_2$) |
| $N^G,N^{G'}$-bis-(2,2,3,3,3-pentafluoro-propyl)homoarginyl | Bph |
| $N^G$-(2,2,3,3,3-pentafluoropropyl)-homoarginyl | Fph |
| $N^G$-ethyl-$N^{G'}$-(2,2,2-tri-fluoroethyl)-homoarginyl | Efh |
| $N^G,N^{G'}$-diethylarginyl | Dea |
| $N^G,N^{G'}$-bis-(2,2,2-trifluoroethyl)-arginyl | Bta, Arg(CH$_2$CF$_3$)$_2$ |
| $N^G$-methylarginyl | Mar, Arg(Me) |

The non-naturally occuring amino acids described herein are prepared by methods well known to those skilled in the art and may be used in either solution phase or solid phase peptide synthesis procedures. (See, for example, Nestor, et.al., **J. Med. Chem.**, **1988**, 31:65).

The following abbreviations exemplify carbohydrate residues which are useful in the glycosylated analogs of this invention.

| Carbohydrate residue | Abbreviation |
|---|---|
| Glucose | Glc |
| Mannose | Mann |
| Fucose | Fuc |
| Rhamnose | Rham |
| Ribose | Rib |
| Maltose | Malt |
| Lactose | Lac |
| Galactose | Gal |
| Arabinose | Ara |
| Sorbitol | Sorb |
| Galactitol | Galol |
| Myo-inositol | Ino |
| *N*-Acetylglucosamine | Glc-NHAc |
| *N*-Acetylgalactosamine | Gal-NHAc |

As used herein, the term "pharmaceutically acceptable salts" refers to salts that retain the desired biological activity of the parent compound and do not impart any undesired toxicological effects. Examples of such salts are:

(a) acid addition salts formed with inorganic acids, for example hydrochloric acid, hydrobromic acid, sulfuric acid, phosphoric acid, nitric acid and the like; and salts formed with organic acids such as, for example, acetic acid, oxalic acid, tartaric acid, succinic acid, maleic acid, fumaric acid, gluconic acid, citric acid, malic acid, ascorbic acid, benzoic acid, tannic acid, pamoic acid, alginic acid, polyglutamic acid, naphthalenesulfonic acids, naphthalenedisulfonic acids, polygalacturonic acid;

(b) base addition salts formed with polyvalent metal cations such as zinc, calcium, bismuth, barium, magnesium, aluminum, copper, cobalt, nickel, cadmium, and the like; or with an organic cation formed from N,N'-dibenzylethylene-diamine or ethylenediamine; or

(c) combinations of (a) and (b), e.g., a zinc tannate salt and the like.

The term "alkyl" refers to a straight or branched chain saturated hydrocarbon radical having from 1 to 8 carbon atoms. Examples of such alkyl groups with the corresponding abbreviation in parentheses, include, but are not limited to, methyl (Me), ethyl (Et), n-propyl (Pr), isopropyl (*i*Pr), butyl (Bu), isobutyl (*i*Bu), *sec*-butyl (*s*Bu), *tert*-butyl (*t*Bu), pentyl (Pe), hexyl (He), cyanomethyl or branched 5 to 8 carbon membered radicals, and the like.

"Fluoroalkyl" refers to an "alkyl" substituted with 1 to 5 fluorine atoms, for example $CF_3$-, $CF_3CH_2$-, $CF_3CF_2CH_2$-, and the like.

A "blocking group" is a moiety which prevents *N*-terminus degradation of a peptide by proteases. Blocking groups are typically acyl or glycosyl groups.

"Acyl" refers to an organic radical derived from a carboxylic acid by the removal of the hydroxyl group. Generally, an acyl group is attached to a terminal amino acid residue on the amine nitrogen.

"N-Ac" refers specifically to the N-acetyl protecting group, i.e., an acetyl group attached to a terminal amino acid residue on the amine nitrogen, in conformance with generally accepted nomenclature.

"Glycosyl" is meant broadly to encompass carbohydrate modifications, however linked to the peptide. Included in this definition are the 1-*O*-glycosylglycolic acids obtained by condensation of the alcohol function of glycolic acid with a sugar residue (e.g. *O*-$\beta$-D-glucosyloxyacetic acid, i.e. $\beta$-D-glc-*O*-$CH_2CO_2$H). An acyl blocking group may also be an Ac-Ser(*O*-glycosyl) or Ac-Thr(*O*-glycosyl) derivative. The acyl linkage may also be through a thiourea or urea functional group. Also included are Amadori rearrangement products, ketose structures obtained by heating an amine with a reducing sugar, referred to herein as ketoglycosyls.

**Preferred Embodiments**

Preferred compounds of this invention are those for which:

A is H, acetyl, or glycosyl;

m is 1; n is 0;

B is D-Arg, hArg($R_1$, $R_2$), D-hArg($R_1$, $R_2$), Arg($R_1$, $R_2$), or D-Arg($R_1$, $R_2$);

C is Gly;

T is Arg, Arg($R_1$) or Arg($R_1$, $R_2$);

E is Hyp or Pro;

F is Thi, Phe, Phe($F_5$), Nal(2) or Phe(Cl);

G is Ser;

I is D-Phe D-Phe(Cl) or D-Tic;

J is Phe, Pro, Oic, Thi, Tyr(Me), Tyr(Et), Phe(Cl), Tic or Phe($F_5$); and

K is Arg, Arg($R_1$) or Arg($R_1$, $R_2$); wherein $R_1$ and $R_2$ are independently Me, Et, or $CH_2CF_3$.

    More preferred are those compounds where:

A is H or acetyl;

$R_1$ and $R_2$ are independently Me or $CH_2CF_3$; and

F is Phe, Phe(Cl) or Thi.

    Specific examples of the preferred compounds include:


Compound Number

1. D-hArg($CH_2CF_3$)$_2$-Arg-Pro-Hyp-Gly-Phe-Ser-D-Phe-Arg(Me);

2. D-hArg($CH_2CF_3$)2-Arg-Pro-Hyp-Gly-Thi-Ser-D-Phe-Thi-Arg(Me);

3. D-hArg($CH_2CF_3$)2-Arg-Pro-Hyp-Gly-Phe($F_5$)-Ser-D-Phe-Phe($F_5$)-Arg(Me);

4. D-hArg($CH_2CF_3$)$_2$-Arg-Pro-Hyp-Gly-Phe-Ser-D-Phe-Tyr(Me)-Arg(Me);

5. D-hArg($CH_2CF_3$)$_2$-Arg-Pro-Hyp-Gly-Nal(2)-Ser-D-Phe-Tyr(Me)-Arg(Me);

6. D-hArg($CH_2CF_3$)$_2$-Arg-Pro-Hyp-Gly-Phe(Cl)-Ser-D-Phe-Phe(Cl)-Arg(Me);

7. D-hArg($CH_2CF_3$)$_2$-Arg-Pro-Hyp-Gly-Phe(Cl)-Ser-D-Phe(Cl)-Tyr(Me)-Arg(Me);

8. D-hArg($CH_2CF_3$)$_2$-Arg-Pro-Hyp-Gly-Phe-Ser-D-Tic-Tyr(Me)-Arg(Me);

9. D-hArg($CH_2CF_3$)$_2$-Arg-Pro-Hyp-Gly-Phe-Ser-D-Tic-Pro-Arg(Me);

10. D-hArg($CH_2CF_3$)$_2$-Arg-Pro-Hyp-Gly-Thi-Ser-D-Tic-Oic-Arg(Me);

11. D-hArg($CH_2CF_3$)$_2$-Arg-Pro-Hyp-Gly-Phe(Cl)-Ser-D-Tic-Pro-Arg(Me);

12. D-hArg($CH_2CF_3$)$_2$-Arg-Pro-Hyp-Gly-Phe(Cl)-Ser-D-Tic-Tyr(Me)-Arg(Me);

13. D-hArg($CH_2CF_3$)$_2$-Arg(Me)-Pro-Hyp-Gly-Phe-Ser-D-Phe-Phe-Arg(Me);

14. D-hArg($CH_2CF_3$)$_2$-Arg(Me)-Pro-Hyp-Gly-Thi-Ser-D-Phe-Thi-Arg(Me);

15. D-hArg($CH_2CF_3$)$_2$-Arg(Me)-Pro-Hyp-Gly-Phe($F_5$)-Ser-D-Phe-Phe($F_5$)-Arg(Me);

16. D-hArg($CH_2CF_3$)$_2$-Arg(Me)-Pro-Hyp-Gly-Phe-Ser-D-Phe-Tyr(Me)-Arg(Me);

17. D-hArg($CH_2CF_3$)$_2$-Arg(Me)-Pro-Hyp-Gly-Nal(2)-Ser-D-Phe-Tyr(Me)-Arg(Me);

18. D-hArg($CH_2CF_3$)$_2$-Arg(Me)-Pro-Hyp-Gly-Phe(Cl)-Ser-D-Phe-Tyr(Me)-Arg(Me);

19. D-hArg($CH_2CF_3$)$_2$-Arg(Me)-Pro-Hyp-Gly-Phe(Cl)-Ser-D-Phe(Cl)-Tyr(Me)-Arg(Me);

20. D-hArg($CH_2CF_3$)$_2$-Arg(Me)-Pro-Hyp-Gly-Phe-Ser-D-Tic-Tyr(Me)-Arg(Me);

21. D-hArg($CH_2CF_3$)$_2$-Arg(Me)-Pro-Hyp-Gly-Phe-Ser-D-Tic-Pro-Arg(Me);

22. D-hArg($CH_2CF_3$)$_2$-Arg(Me)-Pro-Hyp-Gly-Thi-Ser-D-Tic-Oic-Arg(Me);

23. D-hArg($CH_2CF_3$)$_2$-Arg(Me)-Pro-Hyp-Gly-Phe(Cl)-Ser-D-Tic-Pro-Arg(Me);

24. D-hArg($CH_2CF_3$)$_2$-Arg(Me)-Pro-Hyp-Gly-Thi-Ser-D-Tic-Tyr(Me)-Arg(Me);

25. Ac-D-hArg($CH_2CF_3$)$_2$-Arg(Me)-Pro-Hyp-Gly-Phe-Ser-D-Phe-Phe-Arg(Me);

26. Ac-D-hArg($CH_2CF_3$)$_2$-Arg(Me)-Pro-Hyp-Gly-Thi-Ser-D-Phe-Thi-Arg(Me);

27. Ac-D-hArg($CH_2CF_3$)$_2$-Arg(Me)-Pro-Hyp-Gly-Phe($F_5$)-Ser-D-Phe-Phe($F_5$)-Arg(Me);

28. Ac-D-hArg($CH_2CF_3$)$_2$-Arg(Me)-Pro-Hyp-Gly-Phe-Ser-D-Phe-Tyr(Me)-Arg(Me);

29. Ac-D-hArg($CH_2CF_3$)$_2$-Arg(Me)-Pro-Hyp-Gly-Nal(2)-Ser-D-Phe-Tyr(Me)-Arg(Me);

30. Ac-D-hArg($CH_2CF_3$)$_2$-Arg(Me)-Pro-Hyp-Gly-Phe(Cl)-Ser-D-Phe-Phe(Cl)-Arg(Me);

31. Ac-D-hArg($CH_2CF_3$)$_2$-Arg(Me)-Pro-Hyp-Gly-Phe(Cl)-Ser-D-Phe(Cl)-Tyr(Me)-Arg(Me);

32. Ac-D-hArg($CH_2CF_3$)$_2$-Arg(Me)-Pro-Hyp-Gly-Phe-Ser-D-Tic-Tyr(Me)-Arg(Me);

33. Ac-D-hArg($CH_2CF_3$)$_2$-Arg(Me)-Pro-Hyp-Gly-Phe-Ser-D-Tic-Pro-Arg(Me);

34. Ac-D-hArg($CH_2CF_3$)$_2$-Arg(Me)-Pro-Hyp-Gly-Thi-Ser-D-Tic-Oic-Arg(Me);

35. Ac-D-hArg($CH_2CF_3$)$_2$-Arg(Me)-Pro-Hyp-Gly-Phe(Cl)-Ser-D-Tic-Pro-Arg(Me);

36. Ac-D-hArg($CH_2CF_3$)$_2$-Arg(Me)-Pro-Hyp-Gly-Phe(Cl)-Ser-D-Tic-Tyr(Me)-Arg(Me);

37. D-Arg($CH_2CF_3$)$_2$-Arg(Me)-Pro-Hyp-Gly-Phe-Ser-D-Phe-Phe-Arg(Me);

38. D-Arg($CH_2CF_3$)$_2$-Arg(Me)-Pro-Hyp-Gly-Thi-Ser-D-Phe-Thi-Arg(Me);

39. D-Arg($CH_2CF_3$)$_2$-Arg(Me)-Pro-Hyp-Gly-Phe($F_5$)-Ser-D-Phe-Phe($F_5$)-Arg(Me);

40. D-Arg($CH_2CF_3$)$_2$-Arg(Me)-Pro-Hyp-Gly-Phe-Ser-D-Phe-Tyr(Me)-Arg(Me);

41. D-Arg($CH_2CF_3$)$_2$-Arg(Me)-Pro-Hyp-Gly-Nal(2)-Ser-D-Phe-Tyr(Me)-Arg(Me);

42. D-Arg($CH_2CF_3$)$_2$-Arg(Me)-Pro-Hyp-Gly-Phe(Cl)-Ser-D-Phe-Phe(Cl)-Arg(Me);

43. D-Arg($CH_2CF_3$)$_2$-Arg(Me)-Pro-Hyp-Gly-Phe(Cl)-Ser-D-Phe(Cl)-Tyr(Me)-Arg(Me);

44. D-Arg($CH_2CF_3$)$_2$-Arg(Me)-Pro-Hyp-Gly-Phe-Ser-D-Tic-Tyr(Me)-Arg(Me);

45. D-Arg(CH$_2$CF$_3$)$_2$-Arg(Me)-Pro-Hyp-Gly-Phe-Ser-D-Tic-Pro-Arg(Me);

46. D-Arg(CH$_2$CF$_3$)$_2$-Arg(Me)-Pro-Hyp-Gly-Thi-Ser-D-Tic-Oic-Arg(Me);

47. D-Arg(CH$_2$CF$_3$)$_2$-Arg(Me)-Pro-Hyp-Gly-Phe(Cl)-Ser-D-Tic-Pro-Arg(Me);

48. D-Arg(CH$_2$CF$_3$)$_2$-Arg(Me)-Pro-Hyp-Gly-Phe(Cl)-Ser-D-Tic-Tyr(Me)-Arg(Me);

49. Arg(CH$_2$CF$_3$)$_2$-Arg(Me)-Pro-Hyp-Gly-Phe-Ser-D-Phe-Phe-Arg(Me);

50. Arg(CH$_2$CF$_3$)$_2$-Arg(Me)-Pro-Hyp-Gly-Thi-Ser-D-Phe-Thi-Arg(Me);

51. Arg(CH$_2$CF$_3$)$_2$-Arg(Me)-Pro-Hyp-Gly-Phe(F$_5$)-Ser-D-Phe-Phe(F$_5$)-Arg(Me);

52. Arg(CH$_2$CF$_3$)$_2$-Arg(Me)-Pro-Hyp-Gly-Phe-Ser-D-Phe-Tyr(Me)-Arg(Me);

54. Arg(CH$_2$CF$_3$)$_2$-Arg(Me)-Pro-Hyp-Gly-Nal(2)-Ser-D-Phe-Tyr(Me)-Arg(Me);

55. Arg(CH$_2$CF$_3$)$_2$-Arg(Me)-Pro-Hyp-Gly-Phe(Cl)-Ser-D-Phe-Phe(Cl)-Arg(Me);

56. Arg(CH$_2$CF$_3$)$_2$-Arg(Me)-Pro-Hyp-Gly-Phe(Cl)-Ser-D-Phe(Cl)-Tyr(Me)-Arg(Me);

57. Arg(CH$_2$CF$_3$)$_2$-Arg(Me)-Pro-Hyp-Gly-Phe-Ser-D-Tic-Tyr(Me)-Arg(Me);

58. Arg(CH$_2$CF$_3$)$_2$-Arg(Me)-Pro-Hyp-Gly-Phe-Ser-D-Tic-Pro-Arg(Me);

59. Arg(CH$_2$CF$_3$)$_2$-Arg(Me)-Pro-Hyp-Gly-Thi-Ser-D-Tic-Oic-Arg(Me);

60. Arg(CH$_2$CF$_3$)$_2$-Arg(Me)-Pro-Hyp-Gly-Phe(Cl)-Ser-D-Tic-Pro-Arg(Me);

61. Arg(CH$_2$CF$_3$)$_2$-Arg(Me)-Pro-Hyp-Gly-Phe(Cl)-Ser-D-Tic-Tyr(Me)-Arg(Me);

62. Ac-Arg(CH$_2$CF$_3$)$_2$-Arg(Me)-Pro-Hyp-Gly-Phe-Ser-D-Phe-Phe-Arg(Me);

63. Ac-Arg(CH$_2$CF$_3$)$_2$-Arg(Me)-Pro-Hyp-Gly-Thi-Ser-D-Phe-Thi-Arg(Me);

64. Ac-Arg(CH$_2$CF$_3$)$_2$-Arg(Me)-Pro-Hyp-Gly-Phe(F$_5$)-Ser-D-Phe-Phe(F$_5$)-Arg(Me);

65. Ac-Arg(CH$_2$CF$_3$)$_2$-Arg(Me)-Pro-Hyp-Gly-Phe-Ser-D-Phe-Tyr(Me)-Arg(Me);

66. Ac-Arg(CH$_3$CF$_2$)$_2$-Arg(Me)-Pro-Hyp-Gly-Nal(2)-Ser-D-Phe-Tyr(Me)-Arg(Me);

67. Ac-Arg(CH$_2$CF$_3$)$_2$-Arg(Me)-Pro-Hyp-Gly-Phe(Cl)-Ser-D-Phe-Phe(Cl)-Arg(Me);

68. Ac-Arg(CH$_2$CF$_3$)$_2$-Arg(Me)-Pro-Hyp-Gly-Phe(Cl)-Ser-D-Phe(Cl)-Tyr(Me)-Arg(Me);

69. Ac-Arg(CH$_2$CF$_3$)$_2$-Arg(Me)-Pro-Hyp-Gly-Phe-Ser-D-Tic-Tyr(Me)-Arg(Me);

70. Ac-Arg(CH$_2$CF$_3$)$_2$-Arg(Me)-Pro-Hyp-Gly-Phe-Ser-D-Tic-Pro-Arg(Me);

71. Ac-Arg(CH$_2$CF$_3$)$_2$-Arg(Me)-Pro-Hyp-Gly-Thi-Ser-D-Tic-Oic-Arg(Me);

72. Ac-Arg(CH$_2$CF$_3$)$_2$-Arg(Me)-Pro-Hyp-Gly-Phe(Cl)-Ser-D-Tic-Pro-Arg(Me);

73. Ac-Arg(CH$_2$CF$_3$)$_2$-Arg(Me)-Pro-Hyp-Gly-Phe(Cl)-Ser-D-Tic-Tyr(Me)-Arg(Me);

74. D-Arg-Arg-Pro-Hyp-Gly-Phe-Ser-D-Phe-Tyr(Me)-Arg(Me);

75. D-hArg(CH$_2$CF$_3$)$_2$-Arg-Pro-Hyp-Gly-Thi-Ser-D-Tic-Pro-Arg;

76. D-hArg(CH$_2$CF$_3$)$_2$-Arg-Pro-Hyp-Gly-Phe-Ser-D-Phe-Tyr(Et)-Arg(Me);

77. D-hArg(CH$_2$CF$_3$)$_2$-Arg(Me)-Pro-Hyp-Gly-Thi-Ser-D-Tic-Pro-Arg(Me);

78. D-hArg(Et$_2$)-Arg-Pro-Hyp-Gly-Phe-Ser-D-Phe-Phe(F$_5$)-Arg(Me);

79. D-hArg(Et$_2$)-Arg-Pro-Hyp-Gly-Phe-Ser-D-Phe-Pro-Arg(Me);

80. Arg(CH$_2$CF$_3$)$_2$-Arg(CH$_2$CF$_3$)$_2$-Pro-Hyp-Gly-Phe-Ser-D-Phe-Tyr(Me)-Arg(Me$_2$);

81. D-hArg(CH$_2$CF$_3$)$_2$-Arg(Me$_2$)-Pro-Hyp-Gly-Thi-Ser-D-Tic-Oic-Arg(Me$_2$);

82. D-hArg(CH$_2$CF$_3$)$_2$-Arg(Me$_2$)-Pro-Hyp-Gly-Phe-Ser-D-Tic-Pro-Arg(Me$_2$);

83. D-hArg(CH$_2$CF$_3$)$_2$-Arg(Me$_2$)-Pro-Hyp-Gly-Phe-Ser-D-Tic-Tic-Arg(Me$_2$);

84. D-hArg(CH$_2$CF$_3$)$_2$-Arg(Me$_2$)-Pro-Hyp-Gly-Thi-Ser-D-Tic-Oic-Arg;

85. D-hArg(CH$_2$CF$_3$)$_2$-Arg(Me$_2$)-Pro-Hyp-Gly-Phe(Cl)-Ser-D-Tic-Pro-Arg(Me$_2$);

86. D-hArg(CH$_2$CF$_3$)$_2$-Arg(Me$_2$)-Pro-Hyp-Gly-Phe-Ser-D-Tic-Pro-Arg;

87. D-hArg(CH$_2$CF$_3$)$_2$-Arg(Me$_2$)-Pro-Hyp-Gly-Phe(Cl)-Ser-D-Tic-Pro-Arg;

88. D-hArg(CH$_2$CF$_3$)$_2$-Arg(Me$_2$)-Pro-Hyp-Gly-Thi-Ser-D-Tic-Oic-Arg.

89. D-hArg(CH$_2$CF$_3$)$_2$-Arg(Me$_2$)-Pro-Hyp-Gly-Phe-Ser-D-Tic-Oic-Arg(Me$_2$);

90. D-hArg(CH$_2$CF$_3$)$_2$-Arg(Et)-Pro-Hyp-Gly-Phe-Ser-D-Tic-Oic-Arg(Me$_2$);

91. D-hArg(CH$_2$CF$_3$)$_2$-Arg(Me)-Pro-Hyp-Gly-Phe-Ser-D-Tic-Oic-Arg(Me).

In all of the above embodiments, the compounds may also be prepared as pharmaceutically acceptable salts.

## Utility and Assay Procedures

The therapeutic uses of the bradykinin antagonists of this invention include pathological conditions which are known to be mediated by bradykinin or exacerbated by overproduction of bradykinin. These include tissue inflammation and pain that accompany arthritis, asthma, allergy, angina, periodontal disease, rhinitis (viral and allergic), wounds, burns and rashes. Bradykinin antagonists are of value in controlling the overproduction of bradykinin that contributes to early and late phase bronchospasm in asthma, as well as the pain and secretory diarrhea that are characteristic of inflammatory bowel disease. Severe vasodilation and vascular permeability mediated by bradykinin in various shock states (e.g., anaphylactic shock, septic shock, adult respiratory distress syndrome) are reduced or prevented by antagonists of bradykinin. The persistent, dry cough that accompanies antihypertension therapy with ACE inhibitors (presumably a consequence of elevated airway concentrations of bradykinin) may be treated with bradykinin antagonists.

Bioassays used to determine bradykinin antagonist activity are those recognized in the field and include by way of example, the guinea pig ileum receptor binding assay and antagonism of intrarterial bradykinin induced hypotension in rats. The metabolic stability in plasma (mouse, rat, or human) was measured using an HPLC based assay. Mast cell degranulation was assessed by incubation with rat peritoneal mast cells.

Administration

In the practice of this invention an effective amount of a compound of the invention or a pharmaceutical composition thereof is administered to the subject in need of, or desiring, such treatment. These compounds or compositions may be administered by any of a variety of routes depending upon the specific end use, including orally, parenterally (including subcutaneous, intraarticular, intramuscular and intravenous administration), rectally, buccally (including sublingually), transdermally or intranasally. The most suitable route in any given case will depend upon the use, the particular active ingredient, and the subject involved. The compound or composition may also be administered by means of controlled-release, depot implant or injectable formulations as described more fully herein.

In general, for the uses as described in the instant invention, it is expedient to administer the active ingredient in amounts between about 0.1 and 100 $\mu$g/kg body weight, most preferably from about 0.1 to 30 $\mu$g/kg body weight. For human therapy, the active ingredient will be administered preferably in the range of from about 0.1 to about 20-50 $\mu$g/kg/day. This administration may be accomplished by a single administration, by distribution over several applications or by slow release in order to achieve the most effective results. When administered as a single dose, administration will most preferably be in the range of from about 0.1 to 10 $\mu$g/kg.

The exact dose and regimen for administration of these compounds and compositions will necessarily be dependent upon the needs of the individual subject being treated, the type of treatment, and the degree of affliction or need. In general, parenteral administration requires lower dosage than other methods of administration which are more dependent upon absorption.

A further aspect of the present invention relates to pharmaceutical compositions comprising as an active ingredient a compound of the present invention in admixture with a pharmaceutically acceptable, non-toxic carrier. As mentioned above, such compositions may be prepared for use for parenteral (subcutaneous, intraarticular, intramuscular or intravenous) administration, particularly in the form of liquid solutions or suspensions; for oral or buccal administration, particularly in the form of tablets or capsules; or intranasally, particularly in the form of powders, nasal drops or aerosols.

The compositions may conveniently be administered in unit dosage form and may be prepared by any of the methods well-known in the pharmaceutical art, for example as described in Remington's Pharmaceutical Sciences, 17th edition, Mack Publishing Company, Easton, PA., 1985. Formulations for parenteral administration may contain as common excipients sterile water or saline, alkylene glycols such as propylene glycol, polyalkylene glycols such as polyethylene glycol, oils of vegetable origin, hydrogenated naphthalenes and the like. For oral administration, the formulation can be enhanced by the addition of bile salts and also by the addition of acylcarnitines (Am. J. Physiol. 251:332 (1986)). Formulations for nasal administration may be solid and contain as excipients, for example, lactose or dextran, or may be aqueous or oily solutions for administration in the form of nasal drops or metered spray. For buccal administration typical excipients include sugars, calcium stearate, magnesium stearate, pregelatinated starch, and the like.

When formulated for nasal administration the absorption across the nasal mucous membrane is enhanced by surfactant acids, such as for example, glycocholic acid, cholic acid, taurocholic acid, ethocholic acid, desoxycholic acid, chenodesoxycholic acid, dehydrocholic acid, glycodeoxy-cholic acid, and the like. (See, B.H.Vickery, "LHRH and Its Analogs-Contraception and Therapeutic Applications", Pt. 2, B.H. Vickery and J.J. Nestor, Eds., MTP Press, Lancaster, UK, 1987).

One or more surfactant acids or salts, but preferably a single pharmaceutically acceptable acid salt, can be added to the compounds of the instant invention. Suitable pharmaceutically acceptable surfactant salts will be those salts which retain the phenomenon of enhanced peptide absorption, as well as the compound's surfactant characteristics, and which are not deleterious to the subject or otherwise contraindicated. Such salts are for example those salts derived from inorganic bases which include sodium, potassium, lithium, ammonium, calcium, magnesium, ferrous, zinc, copper, manganous, aluminum, ferric, manganic salts and the like. Particularly preferred are the ammonium, potassium, sodium, calcium and magnesium salts. Salts derived from pharmaceutically acceptable organic non-toxic bases include salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-dimethylaminoethanol, 2-diethylaminoethanol, tromethamine, dicyclohexylamine, lysine, arginine, histidine, caffeine, procaine, hydrabamine, choline, betaine, ethylenediamine, glucosamine, methylglucamine, theobromine, purines, piperazine, piperidine, N-ethylpiperidine, polyamine resins and the like. Particularly preferred organic non-toxic bases are isopropylamine, diethylamine, ethanolamine, trimethamine, dicyclohexylamine, choline and caffeine.

The amount of surfactant used for the practice of this invention will be some amount which increases the absorption of the bradykinin analogs over that of other surfactants which also may enhance peptide absorption to a certain degree. It has been found that such an amount is often in the range between 0.2 and 15%, more often 0.2 to 5 percent by weight of the solution. It is preferred that the surfactant be present in an amount between about 0.5 to 4 percent by weight, conveniently about 1 percent by weight, preferably about 2 percent by weight.

It is desirable to deliver the compounds of the present invention to the subject over prolonged periods of time, for example, for periods of one week to one year from a single administration. Various slow release, depot implant or injectable dosage forms may be utilized. For example, a dosage form may contain a pharmaceutically acceptable non-toxic salt of the compound which has a low degree of solubility in body fluids, for example, (a) an acid addition salt with a polybasic acid such as phosphoric acid, sulfuric acid, citric acid, tartaric acid, tannic acid, pamoic acid, alginic acid, polyglutamic acid, naphthalene mono- or di-sulfonic acids, polygalacturonic acid, and the like; (b) a salt with a polyvalent metal cation such as zinc, calcium, bismuth, barium, magnesium, aluminum, copper, cobalt, nickel, cadmium and the like, or with an organic cation formed from e.g., N,N'-dibenzylethylenediamine or ethylenediamine; or (c) combinations of (a) and (b) e.g. a zinc tannate salt. Additionally, the compounds of the present invention or, preferably, a relatively insoluble salt such as those just described, may be formulated in a gel, for example, an aluminum monostearate gel with, e.g. sesame oil, suitable for injection. Particularly preferred salts are zinc salts, zinc tannate salts, pamoate salts, and the like. Another type of slow release depot formulation for injection or implantation would contain the compound or salt dispersed or encapsulated in a slowly degrading, non-toxic, non-antigenic polymer such as a polylactic acid/polyglycolic acid polymer. The compounds or, preferably, relatively insoluble salts such as those described above may also be formulated in cholesterol matrix pellets, or silastomer matrix implants, particularly for use in animals. Additional slow release, depot implant or injectable formulations, e.g. liposomes, are well known in the literature. See, for example, Sustained and Controlled Release Drug Delivery Systems, J. R. Robinson ed., Marcel Dekker, Inc., New York, 1978.

## Synthesis of the Peptides

The compounds of the instant invention may be synthesized by any techniques that are known to those skilled in the peptide art. A summary of the many techniques available may be found in J.M. Stewart and J.D. Young, Solid Phase Peptide Synthesis 2nd edit. Pierce Chemical Co., Rockford, Illinois, 1984, and J. Meienhofer, Hormonal Proteins and Peptides, Vol. 2, p. 46., Academic Press (New York), 1973 for solid phase peptide synthesis and E. Schroder and K. Lubke, The Peptides, Vol. 1, Academic Press (New York), 1965 for classical solution synthesis.

In general, these methods involve the sequential addition of one or more amino acids or suitably protected amino acids to a growing peptide chain. Normally, either the amino or carboxyl group of the first amino acid is protected by a suitable protecting group. The protected or derivatized amino acid can then be either attached to an inert solid support or utilized in solution by adding the next amino acid in the sequence having the complementary (amino or carboxyl) group suitably protected, under conditions suitable for forming the amide linkage. The protecting group is then removed from this newly added amino acid residue and the next amino acid (suitably protected) is then added, and so forth. After all the desired amino acids have been linked in the proper sequence, any remaining protecting groups (and any solid support) are removed sequentially or concurrently, to afford the crude form of the polypeptide. Finally the peptide is desalted and purified chromatographically to yield the final product.

The non-naturally occurring amino acids described herein are prepared by methods well known to those skilled in the art and may be used in either solution phase or solid phase peptide synthesis procedures.

## Preferred Embodiments of Synthesis

A preferred method of preparing the compounds of this invention involves solid phase peptide synthesis.

In this preferred method the α-amino function of the amino acids is protected by an acid-or base-sensitive group. Such protecting groups should have the properties of being stable to the conditions of peptide linkage formation, while being readily removable without destruction of the growing peptide chain or racemization of any of the chiral centers contained therein. Suitable protecting groups are t-butyloxycarbonyl (Boc), benzyloxycarbonyl (Z), o-chlorobenzyloxycarbonyl (Cl-Z), biphenylisopropyloxycarbonyl, t-amyloxycarbonyl, isobornyloxycarbonyl, α,α-dimethyl-3,5-dimethoxybenzyloxycarbonyl, o-nitrophenylsulfenyl, 2-cyano-t-butyloxycarbonyl, 9-fluorenylmethyloxycarbonyl (Fmoc) and the like, especially t-butyloxycarbonyl (Boc).

Particularly preferred side chain protecting groups are, for tyrosine: benzyl (Bzl), o-bromobenzyloxycarbonyl, 2,6-dichlorobenzyl, isopropyl, cyclohexyl, cyclopentyl and acetyl; for serine: benzyl and tetrahydropyranyl; for tryptophan: $N^{IN}$-formyl or no protection.

The C-terminal amino acid is attached to a suitable solid support. Suitable solid supports useful for the above synthesis are those materials which are inert to the reagents and reaction conditions of the stepwise condensation-deprotection reactions, as well as being insoluble in the media used. Suitable solid supports are chloromethylpolystyrene-divinylbenzene polymer, hydroxymethyl-polystyrene-divinylbenzene polymer, and the like, especially chloromethyl-polystyrene-1% divinylbenzene polymer. The attachment to the chloromethyl polystyrene-divinylbenzene type of resin is made by means of the reaction of the $N^{\alpha}$ -protected amino acid, especially the Boc-amino acid, as its cesium, tetramethylammonium, triethylammonium, 1,5-diazabicyclo-[5.4.0]undec-5-ene, or similar salt in ethanol, acetonitrile, N,N-dimethylformamide (DMF), and the like, especially the cesium salt in DMF, with the chloromethyl resin at an elevated temperature, for example between about 40 and 60°C, preferably about 50°C, for from about 12 to 48 hours, preferably about 24 hours. The coupling of successive protected amino acids can be carried out in an automatic polypeptide synthesizer as is well known in the art. The removal of the $N^{\alpha}$ -protecting groups may be performed in the presence of, for example, a solution of trifluoroacetic acid in methylene chloride, hydrogen chloride in dioxane, hydrogen chloride in acetic acid, hydrogen chloride in i-PrOH, or other strong acid solution, preferably 50% trifluoroacetic acid (TFA) in dichloromethane at about ambient temperature. Following neutralization with triethylamine or similar base, each protected amino acid is preferably introduced in approximately 2.5 molar excess and the coupling may be carried out in dichloromethane, dichloromethane/DMF mixtures, DMF and the like, especially in methylene chloride at about ambient temperature. The coupling agent is normally N,N'-dicyclohexylcarbodiimide (DCC) in dichloromethane but may be N,N'-diisopropylcarbodiimide (DIC) or other carbodiimides either alone or in the presence of 1-hydroxybenzotriazole (HBT), N-hydroxysuccinimide, other N-hydroxyimides or oximes. Alternately, protected amino acid active esters (e.g. p-nitrophenyl, pentafluorophenyl and the like) or symmetrical anhydrides may be used.

At the end of the solid phase synthesis the fully protected polypeptide is removed from the resin. Peptides with a free -COOH carboxy-terminus (C-terminus) may be obtained by HF or other strongly acidic deprotection regime; or by saponification. Alternatively, the peptide may be removed from the resin by transesterification, e.g., with methanol, followed by saponification. The protected peptide may be purified at this point by silica gel chromatography. The removal of the side chain protecting groups from the polypeptide is performed by treating the product with, for example, anhydrous liquid hydrogen fluoride in the presence of anisole or other carbonium scavenger, treatment with hydrogen fluoride/pyridine complex, treatment with tris(trifluoroacetyl)boron and trifluoroacetic acid, by reduction with hydrogen and palladium on carbon or polyvinylpyrrolidone, or by reduction with sodium in liquid ammonia, preferably with liquid hydrogen fluoride, and anisole at a temperature between about -10 and +10°C, preferably about 0°C, for between about 15 minutes and 2 hours, preferably about 1 hour. The solution is desalted (e.g. BioRad AG-3 anion exchange resin) and purified by a sequence of chromatographic steps employing any or all of the following types: ion exchange on a weakly basic resin in the acetate form; hydrophobic adsorption chromatography on underivatized polystyrene-divinylbenzene (for example Amberlite XAD); silica gel adsorption chromatography; ion exchange chromatography on carboxymethylcellulose; partition chromatography, e.g., on Sephadex G-25, or countercurrent distribution; high performance liquid chromatography (HPLC), especially reverse phase HPLC on octyl- or octadecylsilyl-silica bonded phase column packing.

Methods for preparing the N-ketoglycosyl, O-glycosylserine acylated, and glycosyloxyacetyl blocked derivatives are well known to those skilled in the art. (See, for example, WO 88/02756 and references therein, the disclosures of which are incorporated by reference herein.)

Another aspect of the present invention relates to a method for preparing compounds of the invention and of the pharmaceutically acceptable salts thereof which process comprises:

removing protecting groups and, optionally, covalently bound solid support from a protected polypeptide to afford a compound of the formula disclosed above or a pharmaceutically acceptable salt thereof, wherein the compound comprises a bradykinin antagonist containing $N^G$-alkylated or fluoroalkylated Arg residues.

## EXAMPLES

The examples which follow are illustrative and not limiting of the invention. General examples of the synthetic routes to the synthesis of the hArg($R_1$) class of non-naturally occurring amino acids are given in Nestor, et.al., **J. Med. Chem.,** **1988,** 31:65 and U.S. Patent 4,667,014 issued May 19, 1987, both of which are incorporated herein by reference.

PREPARATION A

$N^\alpha$-t-Butyloxycarbonyl-$N^G$-methyl-Arginine[Boc-Arg(Me)-OH]

A. S-methyl N-methylisothiouronium hydroiodide salt (1)

To a solution of 1-methyl-2-thiourea (50 g, 0.55 mol) in methanol (250 mL), cooled to 0-5°C was added dropwise methyl iodide (34.6 mL, 0.55 mole). After the addition was completed, the solution was heated at 70°C for 1 hour. The solution was concentrated to ~100 mL. To the concentrated solution, 200 mL $Et_2O$ was added. The solution was cooled and solid appeared. The solid was filtered, washed with ether and dried under vacuum to yield the product as 120 g (94% yield) of white solid.

B. To ornithine hydrochloride (8.4 g, 50 mmol) was added 2 N NaOH until pH 10.65 ± 0.05 was obtained. To the vigorously stirred mixture, was added dropwise at 60°C. a solution of S-methyl N-methylisothiourea in $CH_2Cl_2$, prepared by dissolving 1 (18.56g, 80 mmol) in 4N NaOH and extracting with $CH_2Cl_2$. Positive nitrogen pressure was maintained to blow the mercaptan evolved into an NaOCl trap. The pH (10.65 ± 0.05) was maintained by addition of a concentrated solution of sodium hydroxide. After the addition was completed, the solution was stirred at pH 10.65 at room temperature overnight. The reaction was 80% completed overnight (by amino acid analysis). The reaction mixture was extracted with ethyl acetate to remove the methylurea. The aqueous solution was cooled to 0°C; 100 mL of dioxane was added and di-t-butyl dicarbonate (14.17 g, 65 mmol) in 25 mL dioxane was added dropwise. The mixture was stirred at room temperature overnight, maintaining the pH at 10.00 by the addition of 2N NaOH as needed. The solution was evaporated to half volume to remove dioxane. The aqueous solution was extracted with ethyl acetate, acidified to pH 6.5 with 1N HCl at 0°C and re-extracted with ethyl acetate. The aqueous layer was concentrated and the residue was dissolved in EtOH. Silica Gel (50 g) was added to the solution and evaporated to dryness. The solid was added to a 450 g silica gel column packed in $CH_3CN$ and was eluted with 2L $CH_3CN$, then 2L $CH_3CN/H_2O$ (9/1). The fractions containing pure Boc-Arg(Me)-OH were combined, evaporated, triturated with ether and dried under vacuum. The product was obtained as 3.1 g of glass: 22% yield); mp 99-101°C; $[\alpha]_D^{25}$- 4.68° (1.2% in HOAc).

PREPARATION B

$N^\alpha$-t-butyloxycarbonyl-$N^G$,$N^{G'}$-dimethyl-Arginine (Boc-Arg($Me_2$)-OH).

In a similar manner, ornithine hydrochloride (51 g, 0.3 mol) was converted to Arg($Me_2$)-OH by reaction with S-methyl N,N'-dimethylisothiouronium hydroiodide salt (123 g, 0.49 mol). Arg($Me_2$)-OH was not isolated. The reaction product was reacted with di-t-butyl-dicarbonate (70.8 g, 0.325 mol), followed by purification on a silica gel column to give Boc-Arg($Me_2$)-OH (56.8 g, 62 % yield).

PREPARATION C

$N^\alpha$-t-butyloxycarbonyl-$N^G$,$N^{G'}$-bis-(2,2,2-trifluoroethyl)-D-homoarginine [Boc-D-hArg($CH_2CF_3$)$_2$-OH]

A. Bis(trifluoroethyl)-thiourea

To a vigorously stirred dichloromethane solution containing sodium bicarbonate (37.8 g, 450 mmole) and thiophosgene (8.5 g, 74 mmole) cooled to 0-5°C, was added a solution of trifluoroethylamine hydrochloride (20 g, 148 mmol) in 120 mL water. The reaction mixture was stirred at 0°C for 2 hours and at room temperature overnight. The solid was filtered, washed with water and ether and dried under vacuum. The product was obtained as 14 g (70% yield) of solid.

B. N$^\alpha$-Boc-N$^\epsilon$-Z-D-Lysine methyl ester

To a suspension of N$^\alpha$-Boc-N$^\epsilon$-Z-D-Lysine (100 g, 263 mmol) and NaHCO$_3$ (44.0 g, 523 mmol) in 250 mL of DMF, was added methyl iodide (33.0 mL, 530 mmol) under a N$_2$ atmosphere over 5-10 minutes. The reaction mixture was stirred at room temperature for 36 hours and poured into a mixture of water (1000 mL) and isopropyl acetate (700 mL). The layers were separated. The organic layer was washed with water and brine and dried over anhydrous Na$_2$SO$_4$. The solution was filtered and evaporated under vacuum to give an oil (107 g, 100% yield).

C. N$^\alpha$-Boc-D-Lysine methyl ester hydrochloride

In a 2 liter 3-necked round bottom flask fitted with H$_2$ inlet (under solution), thermometer and overhead stirrer, was placed N$^\alpha$-Boc-N$^\epsilon$-Z-D-Lysine methyl ester (103 g, 261 mmol) in methanol (1000 mL). The solution was degassed. To the solution, 10% Pd (C) (20 g) was added, followed by bubbling of the H$_2$ gas over 3-4 hours. The solution was filtered over Celite®, washed with methanol, and adjusted to pH 4.0 with HCl/ethyl acetate solution. The solution was evaporated under vacuum to yield the product as 76 g (96% yield) of a pale yellow oil.

D. Boc-D-hArg(CH$_2$CF$_3$)$_2$-OH

To a solution of N$^\alpha$-Boc-D-Lysine methyl ester hydrochloride (72.0 g, 243 mmol) in CH$_3$CN (720 mL), was added triethylamine (73.5 g, 727 mmol), bis-trifluorethylthiourea (70.8 g, 290 mmol) and HgCl$_2$ (79.0 g, 290 mmol) at room temperature. The reaction mixture was refluxed for 12 hours to give a thick black solution. The reaction mixture was cooled and the pH of the solution was adjusted to 9 with triethylamine. The reaction mixture was filtered over Celite®, washed with CH$_3$CN, and adjusted to pH 7.0 with 2N HCl. The solution was evaporated under vacuum to give an oil. The oil was redissolved in methanol (1 L) and hydrolyzed with 1 N NaOH at pH 11.4. After completion of hydrolysis, the solution was adjusted to pH 4 and concentrated to dryness. The resulting residue was dissolved in isopropyl acetate (1.0L) and water (0.5L). The aqueous layer was filtered over Celite®, concentrated under vacuum, and adjusted to pH 6.5 with 1 N NaOH. The solution was cooled and a solid resulted. The solid was filtered and dried under vacuum to yield the product as 50 g (46% yield) of solid.

PREPARATION D

N$^\alpha$-t-butyloxycarbonyl-N$^G$,N$^{G'}$-bis-(2,2,2-trifluoroethyl)-D-homoarginine hydrochloride [Boc-D-hArg(CH$_2$CF$_3$)$_2$-Cl] is prepared as follows:

A mixture of 7.33g of benzyl-N$_\alpha$-benzyloxycarbonyl-D-lysinate toluenesulfonate (B. Bezus and L. Zervas, J. Am. Chem. Soc. 83:719 (1961)) and 3.60g. of bis(2,2,2-trifluorodiethyl)thiourea (M. Uher and J. Jendrichovsky, Coll. Czech 38:289 (1973)) in 50 mL CH$_3$CN and 50 mL THF was treated with 2.06g HgCl$_2$ and 3.3 g triethylamine. The reaction mixture was heated to 80-90° C for 8 hours followed by the addition of 20% xs of HgCl$_2$, triethylamine, and the thiourea. Heating was continued for 15 more hours. The reaction was cooled to room temperature, filtered through Celite® and concentrated in vacuo to dryness. The residue was loaded on a silica gel column and eluted with a gradient from CH$_2$Cl$_2$/MeOH (19:1) to CH$_2$Cl$_2$/MeOH (9:1) and then from CH$_2$Cl$_2$/MeOH (9:1) to CH$_2$Cl$_2$/MeOH (4:1). The fractions containing product were detected by thin layer chromatography (TLC), pooled, and concentrated to dryness to yield 7.6g of yellow foam. The foam was repurified on a second silica gel column eluted with a gradient from CH$_2$Cl$_2$/MeOH (9:1) to CH$_2$Cl$_2$/MeOH (4:1) and then isocratic CH$_2$Cl$_2$/MeOH (4:1). The fractions containing product were detected by TLC, pooled, and concentrated to dryness to yield 7.0g of benzyl N$^\alpha$-benzyloxycarbonyl-N$^G$, N$^{G'}$-bis-(2,2,2-trifluoroethyl)-D-homoarginate toluenesulfonate $[\alpha]_D^{25}$ 10.2° (c 1.5% in MeOH).

A 6g portion of the above-named product and 1g 10% Pd/C in 150 mL EtOH was treated with hydrogen gas at atmospheric pressure for 3 hours. An additional 0.4g 10% Pd/C was added and hydrogenation was continued for 3 more hours. The reaction mixture was filtered through celite, concentrated to dryness to give 4g N$^G$,N$^{G'}$-bis-(2, 2,2-trifluoroethyl)-D-homoarginine toluenesulfonate $[\alpha]_D^{25}$-7.76° (0.4% in MeOH).

A solution of this compound (1.96g) in 8 mL 1N NaOH and 8 mL dioxane was treated with 160 mgs MgO and 1.05g di-t-butyldicarbonate at 0° C. The reaction mixture was stirred at 0° C for 1 hour and then at room temperature for 3 hours. The magnesium salt was filtered and the filtrate concentrated under vacuum. The basic solution was washed with anhydrous diethyl ether, then acidified at 0° C with 1N HCl to pH 3.5. The product was extracted from the acidic aqueous solution with ethyl acetate and dried over magnesium sulfate. The drying agent was filtered and the filtrate concentrated to dryness to yield a white foam. The foam was treated with AG-3 Cl$^-$ beads to convert the product to the chloride salt form. 1.4g of N$^\alpha$-t-butyloxycarbonyl-N$^G$,N$^{G'}$-bis-(2,2,2-trifluoroethyl)-D-homoarginine hydrochloride, $[\alpha]_D^{25}$-2.19° (0.5% in MeOH) was isolated.

## PREPARATION E

### Boc-Arg(Me)-O-Resin

Boc-Arg(Me)-OH (8.5 g, 29.7 mmol) was dissolved in 50 mL of ethanol and 10 mL of $H_2O$. The solution was adjusted to pH 7.0 by addition of a 1.5 M solution of cesium carbonate. The mixture was evaporated to dryness and further dried by evaporation of a solution in absolute EtOH (repeated 3 times). The cesium salt was dried under vacuum overnight and used without further purification. The cesium salt was dissolved in 200 mL DMF. To the DMF solution, chloromethyl polystyrene-1%-divinylbenzene resin (20 g, 1.3 meq/g 26 mmol) was added and the suspension was agitated at 50°C for 48 hours. The resin was filtered and washed successively with DMF, a DMF/$H_2O$ mixture (4:1), DMF, $CH_2Cl_2$, EtOH, $CH_2Cl_2$ and dried in vacuo. Amino acid analysis of the resin showed that it had 0.275 meq/g uptake.

## PREPARATION F

### Boc-Arg(Me$_2$)-O-Resin

In a similar manner, Boc-Arg(Me$_2$)-OH (4.8 g, 16 mmol) and chloromethyl resin (10 g, 1.3 meq/g, 13 mmol) were converted to the corresponding Boc-Arg(Me$_2$)-O-Resin (12.8 g, 0.41 meq/g).

## EXAMPLE 1

### Synthesis of compounds of Formula (I)

In the reaction vessel of a Beckman 990 peptide synthesizer was placed 2.0 g (0.54 mmol) of Boc-Arg-(Me)-O-resin; protected amino acids were sequentially added to the resin by means of a standard synthesis program. A typical synthesis program is as described in U.S. Patent 4,667,014 issued May 19, 1987, column 21. Similar synthesis programs for use on other commercially available peptide synthesizers may also be used.

For the preparation of a preferred compound of formula I, the resin was coupled sequentially with a 2.0 to 5.0, preferably 2.0 to 2.5, molar excess of each protected amino acid and N,N'-diisopropylcarbodiimide (DIC). The resin was treated during successive coupling cycles with:

| | |
|---|---|
| 0.40g. | Boc-Tyr(Me)-OH |
| 0.36g. | Boc-D-Phe-OH; |
| 0.40g. | Boc-Ser(Bzl)-OH; |
| 0.36g. | Boc-Phe-OH; |
| 0.24g. | Boc-Gly-OH; |
| 0.43g. | Boc-Hyp(Bzl)-OH; |
| 0.29g. | Boc-Pro-OH; |
| 0.29g. | Boc-Arg(Me)-OH; and |
| 0.61g. | Boc-D-hArg($CH_2CF_3$)$_2$-OH. |

The protected peptide resin was removed from the reaction vessel, filtered and dried in vacuo to yield the protected intermediate. A 2.0 g portion of the peptide resin was deprotected and removed from the resin by treatment with 20 mL of anhydrous liquid HF in the presence of 2 mL of anisole (scavenger) in a Kel-F reaction vessel at 0°C for 1 hour. The HF was evaporated under vacuum and the residue of D-hArg-($CH_2CF_3$)$_2$-Arg(Me)-Pro-Hyp-Gly-Phe-Ser-D-Phe-Tyr(Me)-Arg(Me), as its HF salt, was washed with ether (2x20 mL) and dissolved in $H_2O$ (2x25 mL). Lyophilization of the aqueous solution gave the crude product as white powder.

The crude peptide was purified by preparative high performance liquid chromatography on Vydac $C_{18}$ packing material (2.5x100 cm; 15 micron) using an appropriate gradient of $CH_3CN$ in aqueous $CF_3CO_2H$ (0.1%). The fractions were cut for purity rather than yield (UV monitor) and purity was assessed by analytical HPLC on Vydac analytical columns (5 micron packing). The pooled fractions were lyophilized to yield pure (>95%) D-hArg($CH_2CF_3$)$_2$-Arg(Me)-Pro-Hyp-Gly-Phe-Ser-D-Phe-Tyr(Me)-Arg(Me) as white pow-

der of $[\alpha]_D^{25}$-49.9° (0.37% in $H_2O$).

EXAMPLE 2

Synthesis of compounds of Formula (I)

In the reaction vessel of a Beckman 990 peptide synthesizer was placed 1.0 g (0.41 mmol) of Boc-Arg-($Me_2$)-O-resin; protected amino acids were sequentially added to the resin by means of a standard synthesis program. A typical synthesis program is as described in U.S. Patent 4,667,014 issued May 19, 1987, column 21. Similar synthesis programs for use on other commercially available peptide synthesizers may also be used.

For the preparation of a preferred compound of formula I, the resin was coupled sequentially with a 2.0 to 5.0, preferably 2.0 to 2.5, molar excess of each protected amino acid and N,N'-diisopropylcarbodiimide (DIC). The resin was treated during successive coupling cycles with:

| | |
|---|---|
| 0.44g. | Boc-Oic-OH |
| 0.44g. | Boc-D-Tic-OH; |
| 0.48g. | Boc-Ser(Bzl)-OH; |
| 0.45g. | Boc-Thi-OH; |
| 0.29g. | Boc-Gly-OH; |
| 0.53g. | Boc-Hyp(Bzl)-OH; |
| 0.35g. | Boc-Pro-OH; |
| 0.50g. | Boc-Arg($Me_2$)-OH; and |
| 0.72g. | Boc-D-hArg($CH_2CF_3$)$_2$-OH. |

The protected peptide resin was removed from the reaction vessel, filtered and dried in vacuo to yield the protected intermediate. A 1.5 g portion of the peptide resin was deprotected and removed from the resin by treatment with 15 mL of anhydrous liquid HF in the presence of 1.5 mL of anisole (scavenger) in a Kel-F reaction vessel at 0°C for 1 hour. The HF was evaporated under vacuum and the residue of D-hArg-($CH_2CF_3$)$_2$-Arg($Me_2$)-Pro-Hyp-Gly-Thi-Ser-D-Tic-Oic-Arg($Me_2$), as its HF salt, was washed with ether (2x20 mL) and dissolved in $H_2O$ (2x25 mL). Lyophilization of the aqueous solution gave the crude product as white powder.

The crude peptide was purified by preparative high performance liquid chromatography on Vydac $C_{18}$ packing material (2.5x100 cm; 15 micron) using an appropriate gradient of $CH_3CN$ in aqueous $CF_3CO_2H$ (0.1%). The fractions were cut for purity rather than yield (UV monitor) and purity was assessed by analytical HPLC on Vydac analytical columns (5 micron packing). The pooled fractions were lyophilized to yield pure (>95%) D-hArg($CH_2CF_3$)$_2$-Arg($Me_2$)-Pro-Hyp-Gly-Thi-Ser-D-Tic-Oic-Arg($Me_2$) as white powder of $[\alpha]_D^{25}$-53.48° (0.27% in $H_2O$).

In a similar manner, but using the appropriate sequence of amino acids, were obtained:
1. D-hArg($CH_2CF_3$)$_2$-Arg-Pro-Hyp-Gly-Phe-Ser-D-Phe-Phe-Arg(Me);
2. D-hArg($CH_2CF_3$)2-Arg-Pro-Hyp-Gly-Thi-Ser-D-Phe-Thi-Arg(Me);
3. D-hArg($CH_2CF_3$)2-Arg-Pro-Hyp-Gly-Phe($F_5$)-Ser-D-Phe-Phe($F_5$)-Arg(Me);
4. D-hArg($CH_2CF_3$)$_2$-Arg-Pro-Hyp-Gly-Phe-Ser-D-Phe-Tyr(Me)- Arg(Me);
5. D-hArg($CH_2CF_3$)$_2$-Arg-Pro-Hyp-Gly-Nal(2)-Ser-D-Phe-Tyr(Me)- Arg(Me);
6. D-hArg($CH_2CF_3$)$_2$-Arg-Pro-Hyp-Gly-Phe(Cl)-Ser-D-Phe-Phe(Cl)-Arg(Me);
7. D-hArg($CH_2CF_3$)$_2$-Arg-Pro-Hyp-Gly-Phe(Cl)-Ser-D-Phe(Cl)-Tyr(Me)-Arg(Me);
8. D-hArg($CH_2CF_3$)$_2$-Arg-Pro-Hyp-Gly-Phe-Ser-D-Tic-Tyr(Me)-Arg(Me);
9. D-hArg($CH_2CF_3$)$_2$-Arg-Pro-Hyp-Gly-Phe-Ser-D-Tic-Pro-Arg(Me);
10. D-hArg($CH_2CF_3$)$_2$-Arg-Pro-Hyp-Gly-Thi-Ser-D-Tic-Oic-Arg(Me);
11. D-hArg($CH_2CF_3$)$_2$-Arg-Pro-Hyp-Gly-Phe(Cl)-Ser-D-Tic-Pro-Arg(Me);
12. D-hArg($CH_2CF_3$)$_2$-Arg-Pro-Hyp-Gly-Phe(Cl)-Ser-D-Tic-Tyr(Me)-Arg(Me);
13. D-hArg($CH_2CF_3$)$_2$-Arg(Me)-Pro-Hyp-Gly-Phe-Ser-D-Phe-Phe-Arg(Me);
14. D-hArg($CH_2CF_3$)$_2$-Arg(Me)-Pro-Hyp-Gly-Thi-Ser-D-Phe-Thi-Arg(Me);
15. D-hArg($CH_2CF_3$)$_2$-Arg(Me)-Pro-Hyp-Gly-Phe($F_5$)-Ser-D-Phe-Phe($F_5$)-Arg(Me);
16. D-hArg($CH_2CF_3$)$_2$-Arg(Me)-Pro-Hyp-Gly-Phe-Ser-D-Phe-Tyr(Me)-Arg(Me);
17. D-hArg($CH_2CF_3$)$_2$-Arg(Me)-Pro-Hyp-Gly-Nal(2)-Ser-D-Phe-Tyr(Me)-Arg(Me);
18. D-hArg($CH_2CF_3$)$_2$-Arg(Me)-Pro-Hyp-Gly-Phe(Cl)-Ser-D-Phe-Tyr(Me)-Arg(Me);

19. D-hArg(CH$_2$CF$_3$)$_2$-Arg(Me)-Pro-Hyp-Gly-Phe(Cl)-Ser-D-Phe(Cl)-Tyr(Me)-Arg(Me);

20. D-hArg(CH$_2$CF$_3$)$_2$-Arg(Me)-Pro-Hyp-Gly-Phe-Ser-D-Tic-Tyr(Me)-Arg(Me);

21. D-hArg(CH$_2$CF$_3$)$_2$-Arg(Me)-Pro-Hyp-Gly-Phe-Ser-D-Tic-Pro-Arg(Me);

22. D-hArg(CH$_2$CF$_3$)$_2$-Arg(Me)-Pro-Hyp-Gly-Thi-Ser-D-Tic-Oic-Arg(Me);

23. D-hArg(CH$_2$CF$_3$)$_2$-Arg(Me)-Pro-Hyp-Gly-Phe(Cl)-Ser-D-Tic-Pro-Arg(Me);

24. D-hArg(CH$_2$CF$_3$)$_2$-Arg(Me)-Pro-Hyp-Gly-Thi-Ser-D-Tic-Tyr(Me)-Arg(Me);

25. Ac-D-hArg(CH$_2$CF$_3$)$_2$-Arg(Me)-Pro-Hyp-Gly-Phe-Ser-D-Phe-Phe-Arg(Me);

26. Ac-D-hArg(CH$_2$CF$_3$)$_2$-Arg(Me)-Pro-Hyp-Gly-Thi-Ser-D-Phe-Thi-Arg(Me);

27. Ac-D-hArg(CH$_2$CF$_3$)$_2$-Arg(Me)-Pro-Hyp-Gly-Phe(F$_5$)-Ser-D-Phe-Phe(F$_5$)-Arg(Me);

28. Ac-D-hArg(CH$_2$CF$_3$)$_2$-Arg(Me)-Pro-Hyp-Gly-Phe-Ser-D-Phe-Tyr(Me)-Arg(Me);

29. Ac-D-hArg(CH$_2$CF$_3$)$_2$-Arg(Me)-Pro-Hyp-Gly-Nal(2)-Ser-D-Phe-Tyr(Me)-Arg(Me);

30. Ac-D-hArg(CH$_2$CF$_3$)$_2$-Arg(Me)-Pro-Hyp-Gly-Phe(Cl)-Ser-D-Phe-Phe(Cl)-Arg(Me);

31. Ac-D-hArg(CH$_2$CF$_3$)$_2$-Arg(Me)-Pro-Hyp-Gly-Phe(Cl)-Ser-D-Phe(Cl)-Tyr(Me)-Arg(Me);

32. Ac-D-hArg(CH$_2$CF$_3$)$_2$-Arg(Me)-Pro-Hyp-Gly-Phe-Ser-D-Tic-Tyr(Me)-Arg(Me);

33. Ac-D-hArg(CH$_2$CF$_3$)$_2$-Arg(Me)-Pro-Hyp-Gly-Phe-Ser-D-Tic-Pro-Arg(Me);

34. Ac-D-hArg(CH$_2$CF$_3$)$_2$-Arg(Me)-Pro-Hyp-Gly-Thi-Ser-D-Tic-Oic-Arg(Me);

35. Ac-D-hArg(CH$_2$CF$_3$)$_2$-Arg(Me)-Pro-Hyp-Gly-Phe(Cl)-Ser-D-Tic-Pro-Arg(Me);

36. Ac-D-hArg(CH$_2$CF$_3$)$_2$-Arg(Me)-Pro-Hyp-Gly-Phe(Cl)-Ser-D-Tic-Tyr(Me)-Arg(Me);

37. D-Arg(CH$_2$CF$_3$)$_2$-Arg(Me)-Pro-Hyp-Gly-Phe-Ser-D-Phe-Phe-Arg(Me);

38. D-Arg(CH$_2$CF$_3$)$_2$-Arg(Me)-Pro-Hyp-Gly-Thi-Ser-D-Phe-Thi-Arg(Me);

39. D-Arg(CH$_2$CF$_3$)$_2$-Arg(Me)-Pro-Hyp-Gly-Phe(F$_5$)-Ser-D-Phe-Phe(F$_5$)-Arg(Me);

40. D-Arg(CH$_2$CF$_3$)$_2$-Arg(Me)-Pro-Hyp-Gly-Phe-Ser-D-Phe-Tyr(Me)-Arg(Me);

41. D-Arg(CH$_2$CF$_3$)$_2$-Arg(Me)-Pro-Hyp-Gly-Nal(2)-Ser-D-Phe-Tyr(Me)-Arg(Me);

42. D-Arg(CH$_2$CF$_3$)$_2$-Arg(Me)-Pro-Hyp-Gly-Phe(Cl)-Ser-D-Phe-Phe(Cl)-Arg(Me);

43. D-Arg(CH$_2$CF$_3$)$_2$-Arg(Me)-Pro-Hyp-Gly-Phe(Cl)-Ser-D-Phe(Cl)-Tyr(Me)-Arg(Me);

44. D-Arg(CH$_2$CF$_3$)$_2$-Arg(Me)-Pro-Hyp-Gly-Phe-Ser-D-Tic-Tyr(Me)-Arg(Me);

45. D-Arg(CH$_2$CF$_3$)$_2$-Arg(Me)-Pro-Hyp-Gly-Phe-Ser-D-Tic-Pro-Arg(Me);

46. D-Arg(CH$_2$CF$_3$)$_2$-Arg(Me)-Pro-Hyp-Gly-Thi-Ser-D-Tic-Oic-Arg(Me);

47. D-Arg(CH$_2$CF$_3$)$_2$-Arg(Me)-Pro-Hyp-Gly-Phe(Cl)-Ser-D-Tic-Pro-Arg(Me);

48. D-Arg(CH$_2$CF$_3$)$_2$-Arg(Me)-Pro-Hyp-Gly-Phe(Cl)-Ser-D-Tic-Tyr(Me)-Arg(Me);

49. Arg(CH$_2$CF$_3$)$_2$-Arg(Me)-Pro-Hyp-Gly-Phe-Ser-D-Phe-Phe-Arg(Me);

50. Arg(CH$_2$CF$_3$)$_2$-Arg(Me)-Pro-Hyp-Gly-Thi-Ser-D-Phe-Thi-Arg(Me);

51. Arg(CH$_2$CF$_3$)$_2$-Arg(Me)-Pro-Hyp-Gly-Phe(F$_5$)-Ser-D-Phe-Phe(F$_5$)-Arg(Me);

52. Arg(CH$_2$CF$_3$)$_2$-Arg(Me)-Pro-Hyp-Gly-Phe-Ser-D-Phe-Tyr(Me)-Arg(Me);

54. Arg(CH$_2$CF$_3$)$_2$-Arg(Me)-Pro-Hyp-Gly-Nal(2)-Ser-D-Phe-Tyr(Me)-Arg(Me);

55. Arg(CH$_2$CF$_3$)$_2$-Arg(Me)-Pro-Hyp-Gly-Phe(Cl)-Ser-D-Phe-Phe(Cl)-Arg(Me);

56. Arg(CH$_2$CF$_3$)$_2$-Arg(Me)-Pro-Hyp-Gly-Phe(Cl)-Ser-D-Phe(Cl)-Tyr(Me)-Arg(Me);

57. Arg(CH$_2$CF$_3$)$_2$-Arg(Me)-Pro-Hyp-Gly-Phe-Ser-D-Tic-Tyr(Me)-Arg(Me);

58. Arg(CH$_2$CF$_3$)$_2$-Arg(Me)-Pro-Hyp-Gly-Phe-Ser-D-Tic-Pro-Arg(Me);

59. Arg(CH$_2$CF$_3$)$_2$-Arg(Me)-Pro-Hyp-Gly-Thi-Ser-D-Tic-Oic-Arg(Me);

60. Arg(CH$_2$CF$_3$)$_2$-Arg(Me)-Pro-Hyp-Gly-Phe(Cl)-Ser-D-Tic-Pro-Arg(Me);

61. Arg(CH$_2$CF$_3$)$_2$-Arg(Me)-Pro-Hyp-Gly-Phe(Cl)-Ser-D-Tic-Tyr(Me)-Arg(Me);

62. Ac-Arg(CH$_2$CF$_3$)$_2$-Arg(Me)-Pro-Hyp-Gly-Phe-Ser-D-Phe-Phe-Arg(Me);

63. Ac-Arg(CH$_2$CF$_3$)$_2$-Arg(Me)-Pro-Hyp-Gly-Thi-Ser-D-Phe-Thi-Arg(Me);

64. Ac-Arg(CH$_2$CF$_3$)$_2$-Arg(Me)-Pro-Hyp-Gly-Phe(F$_5$)-Ser-D-Phe-Phe(F$_5$)-Arg(Me);

65. Ac-Arg(CH$_2$CF$_3$)$_2$-Arg(Me)-Pro-Hyp-Gly-Phe-Ser-D-Phe-Tyr(Me)-Arg(Me);

66. Ac-Arg(CH$_3$CF$_2$)$_2$-Arg(Me)-Pro-Hyp-Gly-Nal(2)-Ser-D-Phe-Tyr(Me)-Arg(Me);

67. Ac-Arg(CH$_2$CF$_3$)$_2$-Arg(Me)-Pro-Hyp-Gly-Phe(Cl)-Ser-D-Phe-Phe(Cl)-Arg(Me);

68. Ac-Arg(CH$_2$CF$_3$)$_2$-Arg(Me)-Pro-Hyp-Gly-Phe(Cl)-Ser-D-Phe(Cl)Tyr(Me)-Arg(Me);

69. Ac-Arg(CH$_2$CF$_3$)$_2$-Arg(Me)-Pro-Hyp-Gly-Phe-Ser-D-Tic-Tyr(Me)-Arg(Me);

70. Ac-Arg(CH$_2$CF$_3$)$_2$-Arg(Me)-Pro-Hyp-Gly-Phe-Ser-D-Tic-Pro-Arg(Me);

71. Ac-Arg(CH$_2$CF$_3$)$_2$-Arg(Me)-Pro-Hyp-Gly-Thi-Ser-D-Tic-Oic-Arg(Me);

72. Ac-Arg(CH$_2$CF$_3$)$_2$-Arg(Me)-Pro-Hyp-Gly-Phe(Cl)-Ser-D-Tic-Pro-Arg(Me);

73. Ac-Arg(CH$_2$CF$_3$)$_2$-Arg(Me)-Pro-Hyp-Gly-Phe(Cl)-Ser-D-Tic-Tyr(Me)-Arg(Me);

74. D-Arg-Arg-Pro-Hyp-Gly-Phe-Ser-D-Phe-Tyr(Me)-Arg(Me);

75. D-hArg(CH$_2$CF$_3$)$_2$-Arg-Pro-Hyp-Gly-Thi-Ser-D-Tic-Pro-Arg;

76. D-hArg(CH$_2$CF$_3$)$_2$-Arg-Pro-Hyp-Gly-Phe-Ser-D-Phe-Tyr(Et)-Arg(Me);

77. D-hArg(CH$_2$CF$_3$)$_2$-Arg(Me)-Pro-Hyp-Gly-Thi-Ser-D-Tic-Pro-Arg(Me);

78. D-hArg(Et$_2$)-Arg-Pro-Hyp-Gly-Phe-Ser-D-Phe-Phe(F$_5$)-Arg(Me);

79. D-hArg(Et$_2$)-Arg-Pro-Hyp-Gly-Phe-Ser-D-Phe-Pro-Arg(Me);

80. Arg(CH$_2$CF$_3$)$_2$-Arg(CH$_2$CF$_3$)$_2$-Pro-Hyp-Gly-Phe-Ser-D-Phe-Tyr(Me)-Arg(Me);

81. D-hArg(CH$_2$CF$_3$)$_2$-Arg(Me$_2$)-Pro-Hyp-Gly-Thi-Ser-D-Tic-Oic-Arg(Me$_2$);

82. D-hArg(CH$_2$CF$_3$)$_2$-Arg(Me$_2$)-Pro-Hyp-Gly-Phe-Ser-D-Tic-Pro-Arg(Me$_2$);

83. D-hArg(CH$_2$CF$_3$)$_2$-Arg(Me$_2$)-Pro-Hyp-Gly-Phe-Ser-D-Tic-Tic-Arg(Me$_2$);

84. D-hArg(CH$_2$CF$_3$)$_2$-Arg(Me$_2$)-Pro-Hyp-Gly-Thi-Ser-D-Tic-Oic-Arg;

85. D-hArg(CH$_2$CF$_3$)$_2$-Arg(Me$_2$)-Pro-Hyp-Gly-Phe(Cl)-Ser-D-Tic-Pro-Arg(Me$_2$);

86. D-hArg(CH$_2$CF$_3$)$_2$-Arg(Me$_2$)-Pro-Hyp-Gly-Phe-Ser-D-Tic-Pro-Arg;

87. D-hArg(CH$_2$CF$_3$)$_2$-Arg(Me$_2$)-Pro-Hyp-Gly-Phe(Cl)-Ser-D-Tic-Pro-Arg;

88. D-hArg(CH$_2$CF$_3$)$_2$-Arg(Me$_2$)-Pro-Hyp-Gly-Thi-Ser-D-Tic-Oic-Arg.

89. D-hArg(CH$_2$CF$_3$)$_2$-Arg(Me$_2$)-Pro-Hyp-Gly-Phe-Ser-D-Tic-Oic-Arg(Me$_2$);

90. D-hArg(CH$_2$CF$_3$)$_2$-Arg(Et)-Pro-Hyp-Gly-Phe-Ser-D-Tic-Oic-Arg(Me$_2$);

91. D-hArg(CH$_2$CF$_3$)$_2$-Arg(Me)-Pro-Hyp-Gly-Phe-Ser-D-Tic-Oic-Arg(Me).

In all of the above embodiments, the compounds may also be prepared as pharmaceutically acceptable salts. $[\alpha]_D^{25}$'s were determined for selected compounds in H$_2$O.

| Compounds in H$_2$O | $[\alpha]_D^{25}$ at concentration (wt. %) | |
|---|---|---|
| 4 | −42.63° | 0.19% |
| 76 | −48.07° | 0.28 |
| 18 | −52.01° | 0.37 |
| 77 | −52.98° | 0.3 |
| 78 | −46.7° | 0.14 |
| 79 | −69.3° | 0.3 |
| 52 | −44.58° | 0.2 |
| 80 | −52.66° | 0.14 |

### EXAMPLE 3

#### Glycosylated derivatives

N$^{\alpha}$-$\beta$-deoxyfructosyl-D-hArg(CH$_2$CF$_3$)$_2$-Arg(Me)-Pro-Hyp-Gly-Phe-Ser-D-Phe-Phe-Arg(Me).

A. A solution of 0.72g of D-hArg(CH$_2$CF$_3$)$_2$-Arg(Me)-Pro-Hyp-Gly-Phe-Ser-D-Phe-Phe-Arg(Me) in 25mL of MeOH/HOAc(9:1) is treated with 1.8g of D-glucose and heated at 70°C for 3 hours. The solution is concentrated by evaporation in vacuo, diluted with MeOH and the product precipitated with diethylether. The product is purified by chromatography on silica gel (CH$_2$Cl$_2$/MeOH/HOAc; 8:1:1).

The title compound is precipitated with 30mL Et$_2$O, washed by centrifugation/decantation (Et$_2$O), and dried to white powder. The purified product is obtained by silica gel chromatography (CH$_2$Cl$_2$/MeOH/HOAc; 7:2:1).

B. In a similar manner, but substituting for glucose:

**D**-(+)-maltose, **D**-(+)-galactose, **D**-ribose, the corresponding N$^{\alpha}$-($\alpha$-**D**-Glucopyranosyl-(1-4)-1-deoxyfructosyl), N$^{\alpha}$-(deoxysorbosyl), or N$^{\alpha}$-(deoxyribulosyl) analogs, respectively, may be obtained.

In a similar manner, are obtained the corresponding N$^{\alpha}$, N$^{\epsilon}$-di(deoxyfructosyl), N$^{\alpha}$, N$^{\epsilon}$-di-(deoxysorbosyl), and N$^{\alpha}$, N$^{\epsilon}$-di(deoxyribulosyl) analogs.

### EXAMPLE 4

#### Acylated derivatives

A. A solution of 0.8g of D-hArg(CH$_2$CF$_3$)$_2$-Arg(Me)-Pro-Hyp-Gly-Phe-Ser-D-Tic-Pro-Arg(Me) is treated with 0.3g of 2,3,4,6-tetra-O-acetyl-$\beta$-D-glucosylisothiocyanate at 25°C for 1 hour. The crude product is recovered by concentration in vacuo and trituration with Et$_2$O.

The pure product is obtained by reverse phase chromatography using a 2.5 x 100cm column of Vydac and a gradient of 10-45% CH$_3$CN (0.04M in NH$_4$OAc at pH4.5).

In a similar fashion but substituting 2,3,4,6-tetra-O-acetyl-$\beta$-D-glucosyl-isocyanate is obtained the corresponding $\beta$-D-glucosylcarbamoyl analog.

B. A solution of 0.8g of D-hArg($CH_2CF_3$)$_2$-Arg(Me)-Pro-Hyp-Gly-Phe-Ser-D-Tic-Pro-Arg(Me) is treated with 0.32g of 2,3,4,6-tetra-O-acetyl-O-$\beta$-D-glucosyloxyacetic acid in 2 mL of DMF (the acid is pretreated with 45mg of HBT and 45mg of N,N'-dicyclohexylcarbodiimide). After 3 hours at 25°C, the precipitate of DCU is filtered and the crude intermediate precipitated by the addition of 25 mL Et$_2$O. The O-acetyl protecting groups are removed by the addition of a catalytic amount of NaOMe to a solution of the intermediate in 40mL of MeOH. The product may be obtained by trituration and centrifugation/decantation (Et$_2$O).

EXAMPLE 5

Guinea Pig Ileum

Bradykinin Receptor Binding Assay

The bradykinin analogs were assayed using a cell-free membrane receptor binding assay described by Innis et al *Proc. Natl. Acad. Sci. USA* 1981, 78:2630. Male Hartley guinea pigs (400-500g, Charles River) were euthanized by $CO_2$ asphyxiation. A 5 cm section of the distal ileum was removed, rinsed in cold saline and homogenized in 20 volumes of cold 20 mM TES buffer, pH 6.8, 1mM 1,10-phenanthroline using a Tissumizer (Tekmar) at 75% maximum speed for 12 seconds. The homogenate was centrifuged at 15,000 X g for 25 minutes at 4°C. The crude membrane pellet was washed once in 20 volumes of fresh homogenizing buffer and resuspended in assay buffer (25 mM TES buffer, pH 6.8, 1 mM dithiothreitol, 1 mM 1,10-phenanthroline, 1 $\mu$M captopril, 0.014% bacitracin and 0.1% bovine serum albumin) at 100 mL buffer per gram issue. The membrane preparation was quick-frozen in liquid N$_2$ and stored at -80°C.

The reaction mixture for the receptor binding assay contained bradykinin analog (1-1000 nM), 100 pM [$^3$H]bradykinin (88 Ci/mmol) and 200 $\mu$l membrane preparation in a total volume of 500 $\mu$l assay buffer. Reactions were incubated at room temperature for 90 minutes and membrane-bound [$^3$H]bradykinin was isolated by filtration through PEI-coated glass fiber filters. The filters were dried and counted for radioactivity in a liquid scintillation counter. Specific binding was calcuated by subtracting the nonspecific binding (determined in a parallel set of assays in the presence of 1 $\mu$M unlabeled bradykinin) from the total radioactivity bound to the filters. All incubations were performed in duplicate and the data expressed as % reduction in the specific binding of [$^3$H]bradykinin. The IC$_{50}$ (the concentration of analog that reduced the binding of 100 pM [$^3$H]bradykinin by 50%) was determined graphically from a plot of % reduction vs. log concentration. For comparison, the IC$_{50}$ for bradykinin is 0.1 to 0.2 nM

The IC$_{50}$ values of some of the compounds of this invention are as follows:

| Compound No. | IC$_{50}$(nM) |
|---|---|
| 74 | 30 |
| 75 | 1 |
| 21 | 2.2 |
| 22 | 0.05 |
| 91 | 0.05 |
| 89 | 0.07 |
| 90 | 0.04 |
| 81 | 0.15 |

Other compounds of the invention have similar IC$_{50}$ values.

EXAMPLE 6

Plasma Stability In Vitro

The ability of bradykinin antagonists to resist proteolysis was determined by measuring the half-life of the analogs in plasma using HPLC. Rat blood was collected into heparin-containing tubes and centrifuged at 2000 X g for 10 minutes at 4°C to separate the plasma. Plasma samples were supplemented with bradykinin analogs (60 $\mu$M) and incubated at 37°C. At various times during incubation, an aliquot was removed and the reaction was stopped with 10% trifluoroacetic acid (TFA). The reaction mixture was applied to a 1 mL $C_{18}$ solid-phase extraction column (Baker) preconditioned with methanol and 10% TEA in $H_2O$. The analog was eluted with 3 x 100 $\mu l$ of 15% acetonitrile ($CH_3CN$), 85% $H_2O$ containing 0.2% TFA. The eluent was dried in a Speed-Vac concentrator (Savant) and redissolved in 300 $\mu L$ of mobile phase consisting of various proportions of $CH_3CN$ and $H_2O$ containing 0.2% TEA depending on the analog. The sample was injected onto a Pecosphere® (Perkin-Elmer) 3 $\mu m$ $C_{18}$ cartridge column (3.3 x 0.46 cm) and eluted with mobile phase at a flow rate of 1 ml/min using a Waters HPLC system. The analog was monitored by uv absorption at 210 nm and the results analyzed with a Hewlett-Packard integrator. Peak height was used to quantitate the amount of analog present in the sample. Plasma degradation followed first-order kinetics and the $t_{1/2}$ (the time required for half the sample to be degraded) was determined graphically from a plot of log % analog remaining vs. incubation time. Compounds 1, 74, 22 and 91 have half-lives in excess of 120 minutes in mouse and human plasma. For comparison, the half-life of bradykinin in mouse, rat or human plasma is less than 5 minutes.

EXAMPLE 7

Protection Against Bradykinin-induced Hypotension in Rats

Male Sprague-Dawley rats (200-300 g, Charles River) were anesthetized with 40 mg/kg ip sodium pentobarbital. The left femoral artery and vein were fitted with polyethylene cannulas (Intramedic PE-50) and the left carotid artery was fitted with a PE-20 cannula. The carotid cannula was used for the administration of bradykinin and bradykinin analogs. The femoral arterial cannula was connected to a blood pressure transducer (Statham) and Beckman dynograph for recording systolic and diastolic pressure. The femoral venous cannula was used to administer maintenance anesthesia. In some studies, the right femoral vein was cannulated and used to infuse bradykinin and analogs to compare the venous and arterial routes of administration. All cannulas were filled with heparinized (40 U/mL) saline to maintain patency.

Rats were placed on a 37°C water blanket and allowed to reach a stable anesthetic plane characterized by a mean arterial blood pressure of $\geq$90 mm Hg and a positive response to a toe pinch. Each rat was given two consecutive injections of bradykinin (0.4 $\mu$g/kg in 0.5 mL/kg) via the left carotid cannula to establish a standard hypotensive response (35-55 mm Hg drop in mean arterial blood pressure). The interval between injections was 5 minutes. The hypotensive response to bradykinin is very short-lived (less than 3 minutes) so that blood pressure returns to baseline levels between injections. Rats do not become sensitized (tachyphylactic) to repetitive doses of bradykinin under the conditions described above up to at least 7 consecutive doses of bradykinin. Co-administration of compound 4 (50-200 nmol/kg) with bradykinin reduced or prevented the drop in blood pressure demonstrating protection against bradykinin-induced hypotension.

EXAMPLE 8

Mast Cell Degranulation Assay

Mixed peritoneal cells were recovered from 3 or 4 male Sprague-Dawley rats (350 g, Iffa-Credo, France) by intraperitoneal injection of 10 mL of 0.9% NaCl (containing 50 $\mu$g/mL heparin). After gentle massage of the abdominal cavity for 1 minute, the peritoneal cells were removed, pooled and centrifuged for 5 minutes at 300 g. After three rinses in Krebs-Ringer Buffer (KRB: 141.9 mM NaCl; 4.7 mM KCl; 1.0 mM $CaCl_2$, 11.2 mM $MgSO_4$; 2.5 mM $Na_2HPO_4$; 0.6 mM $KH_2PO_4$), the cells were counted under a microscope and diluted in the appropriate volume of KRB to achieve a cell density of about 2 x $10^6$ cells per mL. Aliquots of 0.5 mL were prewarmed with 0.4 mL $Ca^{++}$-free KRB for 5 minutes at 35°C and 0.1 mL of the appropriate solution of the drugs tested was added or its vehicle alone. 15 minutes later, the reaction was stopped by the addition of 2.5 mL ice-cold KRB and chilling on ice.

After centrifugation of the cell suspension, the histamine content of the supernatant was assayed fluorimetrically by the method of Shore et al., (**Immunol. 1959, 127**:182) omitting the extraction procedure (Excitation 365 nm; Emission 450 nm). None of the reagents fluoresced with o-Phthaldialdehyde at the concentrations used in the experiments.

The total histamine content of the cell suspension was determined after sonication (2 minutes - 5 second pulse frequency).

The spontaneous histamine release was subtracted from all the values measured.

Compounds of the invention, for example, compounds 89 and 90, show reduced histamine release relative to prior art bradykinin antagonists (EC50 values for compounds 89 and 90 are 312 and 216 $\mu$g/mL respectively).

EXAMPLE 9

Mouse Bradykinin Writhing Test

The bradykinin analogs were assayed for analgesic activity using the method described by T. Walter et al. in **Agents and Actions** **1989**, 27:375. Male CD-1 mice (20-30 g, Charles River) were pretreated with prostaglandin $E_2$ (1 mg/kg intraperitoneally) at 20 minutes before challenge with bradykinin (0.5 mg/kg intraperitoneally). The number of writhes per mouse in a test group of eight animals was determined for two minutes immediately following bradykinin injection. Vehicle or bradykinin analogs were administered intraperitoneally at two minutes prior to bradykinin. Compounds of this invention inhibit the writhing response at doses substantially lower than those required by prior art bradykinin antagonists.

EXAMPLE 10

Carrageenan-induced Rat Paw Edema Assay

The anti-inflammatory action of bradykinin antagonists was evaluated using the carrageenan-induced rat paw edema assay. Eight 80-100g female rates were treated with test material as follows. At zero hour animals were administered dorsally 0.5 ml of the compounds 89 and 81. A saline vehicle was used as a positive control. At +1 hours 0.05 ml of a 1% solution (in 0.9% saline) of carrageenan (Type IV lambda) from SIGMA was injected sub plantar into the ventral side of the right hind paw to induce inflammation. At +4 hours (3 hours after carrageenan injection) paw thickness was measured using dial thickness gauge calipers.

Compounds 89 and 81 showed 50% inhibition of inflammation in the range of 0.01 to 0.001 $\mu$g/mL.

EXAMPLE 11 - TOXICITY

In the above Example 10, no toxic effects were observed with the compounds of this invention.

**Claims**

1. A compound of the formula:

   A-(B)$_m$-(C)$_n$-T-E-E-C-F-G-I-J-K

   wherein:

   A is H, acyl or glycosyl;
   B is D-Arg, Arg(R$_1$), D-Arg(R$_1$), hArg(R$_1$), D-hArg(R$_1$), Arg(R$_1$,R$_2$), D-Arg(R$_1$,R$_2$), hArg(R$_1$,R$_2$), or D-hArg(R$_1$,R$_2$), where R$_1$ is alkyl or fluoroalkyl and R$_2$ is cyano, alkyl, or fluoroalkyl;
   C is $\beta$-Ala, Gly, or aza-Gly;
   T is Arg or B;
   E is Hyp or Pro;
   F is Nal(1), Nal(2), Phe, Phe(Cl), Phe(F$_5$), Thi, Trp, or Tyr(OMe);
   G is Gly, D-Phe, Ser, or D-Thi;
   I is D-Ala, D-Dic, D-Hyp, D-Nal(1), D-Nal(2), D-Ohc, D-Oic, D-Pal(3), D-Phe, D-Phe(Cl), D-Pip, D-Pro, D-Thi, D-Thp, D-Tic, D-Trp, D-Tyr, D-Tyr(Me), D-$\alpha$MeNal(2), D-$\alpha$MePhe, or D-$\alpha$Mephe(Cl);
   J is Dic, Hyp, Nal(1), Nal(2), Ohc, Oic, Phe, Phe(F$_5$), Phe(Cl), Pip, Pro, Thi, Thp, Tic, Tyr(Me), Tyr(Et), $\alpha$MeNal(2), $\alpha$MePhe, or $\alpha$MePhe(Cl);
   K is Arg or B;
   m is 1, 2, 3, 4, or 5; and
   n is 0, 1, or 2;
   or a pharmaceutically acceptable salt thereof.

2. A compound of Claim 1 wherein:
   A is H or acetyl;

m is 1; n is 0;

B is D-Arg, hArg($R_1$,$R_2$), D-hArg($R_1$,$R_2$), Arg($R_1$,$R_2$), or D-Arg($R_1$,$R_2$);

C is Gly;

T is Arg, Arg($R_1$) or Arg($R_1$,$R_2$);

E is Hyp or Pro;

F is Thi, Phe, Phe($F_5$), Nal(2), or Phe(Cl);

G is Ser;

I is D-Phe,D-Phe(Cl), or D-Tic;

J is Oic, Phe, Pro, Tic, Thi, Phe(Cl), Tyr(Et), Phe($F_5$), or Tyr(Me); and

K is Arg, Arg($R_1$) or Arg($R_1$, $R_2$);

$R_1$ and $R_2$ are independently Me, Et, or $CH_2CF_3$; or a pharmaceutically acceptable salt thereof.

3. A compound of Claim 2 wherein B is Arg($R_1$,$R_2$), D-Arg($CH_2CF_3$)$_2$, hArg($CH_2CF_3$)$_2$, or D-hArg($CH_2CF_3$)$_2$, or a pharmaceutically acceptable salt thereof.

4. A compound of Claim 3 wherein K is Arg(Me), Arg(Me$_2$), or Arg, or a pharmaceutically acceptable salt thereof.

5. A compound of Claim 4 wherein F is Phe, Phe(Cl), or Thi; $R_1$ and $R_2$ are independently Me, Et or $CH_2CF_3$; or a pharmaceutically acceptable salt thereof.

6. A compound of Claim 5 wherein A is H; B is D-hArg($CH_2CF_3$)$_2$; T is Arg(Me$_2$); E-E is Pro-Hyp; I is D-Tic; J is Oic, Pro, or Tic; and K is Arg(Me$_2$) or Arg, or a pharmaceutically acceptable salt thereof.

7. A compound of Claim 6 wherein F is Phe, J is Pro, and K is Arg(Me$_2$), or a pharmaceutically acceptable salt thereof.

8. A compound of Claim 6 wherein F is Phe, J is Tic, and K is Arg(Me$_2$), or a pharmaceutically acceptable salt thereof.

9. A compound of Claim 6 wherein F is Thi, J is Oic, and K is Arg(Me$_2$), or a pharmaceutically acceptable salt thereof.

10. A compound of Claim 6 wherein F is Phe(Cl), J is Pro, and K is Arg(Me$_2$), or a pharmaceutically acceptable salt thereof.

11. A compound of Claim 6 wherein F is Phe, J is Pro, and K is Arg, or a pharmaceutically acceptable salt thereof.

12. A compound of Claim 6 wherein F is Phe(Cl), J is Pro, and K is Arg, or a pharmaceutically acceptable salt thereof.

13. A compound of Claim 6 wherein F is Thi, J is Oic, and K is Arg, or a pharmaceutically acceptable salt thereof.

14. A compound of Claim 6 wherein F is Thi, J is Tic, and K is Arg, or a pharmaceutically acceptable salt thereof.

15. A compound of Claim 6 wherein F is Phe, J is Oic, and K is Arg(Me$_2$), or a pharmaceutically acceptable salt thereof.

16. A compound of Claim 5 wherein A is H; B is D-hArg($CH_2CF_3$)$_2$; T is Arg(Et); -E-E- is -Pro-Hyp-; F is Phe; I is D-Tic; J is Oic; and K is Arg(Me$_2$), or a pharmaceutically acceptable salt thereof.

17. A compound of Claim 5 wherein A is H; B is D-hArg($CH_2CF_3$)$_2$; T is Arg or Arg(Me); -E-E- is -Pro-Hyp- F is Phe or Thi; I is D-Phe or D-Tic; J is Tyr(Me), Pro or Oic; and K is Arg(Me) or a pharmaceutically acceptable salt thereof.

22

**18.** A compound of Claim 17 wherein T is Arg, F is Phe, I is D-Phe and J is Tyr(Me), or a pharmaceutically acceptable salt thereof.

**19.** A compound of Claim 17 wherein T is Arg, F is Thi, I is D-Tic and J is Oic, or a pharmaceutically acceptable salt thereof.

**20.** A compound of Claim 17 wherein T is Arg(Me), F is Phe, I is D-Phe and J is Tyr(Me), or a pharmaceutically acceptable salt thereof.

**21.** A compound of Claim 17 wherein T is Arg(Me), F is Phe, I is D-Tic and J is Pro, or a pharmaceutically acceptable salt thereof.

**22.** A compound of Claim 17 wherein T is Arg(Me), F is Thi, I is D-Tic and J is Oic, or a pharmaceutically acceptable salt thereof.

**23.** A compound of Claim 17 wherein T is Arg(Me), F is Phe, I is D-Tic, and J is Oic, or a pharmaceutically acceptable salt thereof.

**24.** A pharmaceutical composition comprising a compound of any of Claims 1-23, or a pharmaceutically acceptable salt thereof, in admixture with at least one pharmaceutically acceptable excipient.

**25.** A compound according to any one of Claims 1-23 for use as a pharmaceutical.

**26.** The use of a compound of any of Claims 1-23, or a pharmaceutically acceptable salt thereof in the preparation of a medicament for the treatment of trauma or pathological conditions in a human being induced or mediated by bradykinin, in particular wherein the condition to be treated is osteoarthritis or rheumatoid arthritis, of allergic or viral rhinitis.

**27.** A process for preparing a compound according to Claim 1 which process comprises removing protecting groups and, optionally, covalently bound solid support from a protected polypeptide to afford a compound of Formula (I) or a salt thereof; or coupling together in the required sequence two fragments of the desired compound of Formula (I); or
    (a) converting a compound of Formula (I) to a pharmaceutically acceptable salt, or
    (b) converting a salt of a compound of Formula (I) to a pharmaceutically acceptable salt, or
    (c) converting a salt of a compound of Formula (I) to a free polypeptide of Formula (I).

| | DOCUMENTS CONSIDERED TO BE RELEVANT | | EP 91114248.7 |
|---|---|---|---|
| **Category** | Citation of document with indication, where appropriate, of relevant passages | Relevant to claim | **CLASSIFICATION OF THE APPLICATION (Int. Cl.5)** |
| A | EP - A2 - 0 370 453 (HOECHST) * Claims * | 1,26, 27 | C 07 K 7/18 A 61 K 37/42 C 07 K 1/04 C 07 K 1/06 |
| A | WO - A1 - 89/09 231 (ADMINISTRATORS OF THE TULANE EDUCATIONAL FUND) * Claims 1,5 * | 1,26, 27 | |
| D,A | WO - A1 - 89/01 781 (STEWART J.M. et al.) * Claims 1,35 * | 1,26 | |
| A | WO - A1 - 86/07 263 (STEWART J.M. et al.) * Claims * | 1,26 | |
| A | CHEMICAL ABSTRACTS, vol. 97, no. 25, December 20, 1982, Columbus, Ohio, USA J. CHRISTIANSEN et al. "Amino-acids and peptides. Part 46. Synthesis of bradykinin analogs modified in the vicinity of the carboxy-group" pages 901-902, right and left column, abstract-no. 216 658w & J. Chem. Soc., Perkin Trans. 1, 1982, (5), 1229-37 | 1,27 | **TECHNICAL FIELDS SEARCHED (Int. Cl.5)** C 07 K A 61 K |
| A | CHEMICAL ABSTRACTS, vol. 85, no. 13, September 27, 1976, Columbus, Ohio, USA H. AROLD et al. "Peptides. XXVI. Synthesis of (8-phenylglycine)brady- kinin in combination with nor- and homoarginine in | 1,27 | |

The present search report has been drawn up for all claims

| Place of search VIENNA | Date of completion of the search 06-11-1991 | Examiner AUGUSTIN |
|---|---|---|

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

   .............................................................
& : member of the same patent family, corresponding
   document

EPO FORM 1503 03.82 (P0401)

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| | position 9" <br> page 669, left column, <br> abstract-no. 94 672a <br>      & J. Prakt. Chem. 1976, 318 <br>      (3), 441-9 <br> -- | | |
| A | CHEMICAL ABSTRACTS, vol. 85, <br> no. 17, October 25, 1976, <br> Columbus, Ohio, USA <br> H. AROLD <br> "Peptides. XXIV. Synthesis <br> of (8-alpha-amino-gamma- <br> -phenylbutyric acid)brady- <br> kinin in combination with <br> nor- and homoarginine in <br> position 9" <br> page 681, right column, <br> abstract-no. 124 341m <br>      & J. Prakt. Chem. 1976, 318 <br>      (3), 420-8 <br> -- | 1,27 | |
| A | CHEMICAL ABSTRACTS, vol. 75, <br> no. 5, August 2, 1971, <br> Columbus, Ohio, USA <br> H. AROLD <br> "Peptides. XVII. Synthesis of <br> homoarginine bradykinins" <br> page 567, left column, <br> abstract-no. 36 640w <br>      & J. Prakt. Chem. 1970, 312 <br>      (6), 1161-74 <br> -- | 1 | TECHNICAL FIELDS SEARCHED (Int. Cl.5) |
| D,A | CHEMICAL ABSTRACTS, vol. 103, <br> no. 13, September 30, 1985, <br> Columbus, Ohio, USA <br> R.J. VAVREK et al. <br> "Competitive antagonists of <br> bradykinin" <br> page 69, right column, <br> abstract-no. 98 853y | 1,27 | |

The present search report has been drawn up for all claims

| Place of search | Date of completion of the search | Examiner |
|---|---|---|
| VIENNA | 06-11-1991 | AUGUSTIN |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
........................................................................
& : member of the same patent family, corresponding
    document

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| | & Peptides (Fayetteville, N.Y.) 1985, 6(2), 161-4 -- | | |
| A | CHEMICAL ABSTRACTS, vol. 105, no. 21, November 24, 1986, Columbus, Ohio, USA R.J. VAVREK et al. "Development and modification of competitive antagonists of bradykinin" page 85, left and right column, abstract-no. 184 127n & Pept.: Struct. Funct., Proc. Am. Pept. Symp., 9th 1985, 655-8 ---- | 1 | |
| | | | TECHNICAL FIELDS SEARCHED (Int. Cl.5) |

The present search report has been drawn up for all claims

| Place of search | Date of completion of the search | Examiner |
|---|---|---|
| VIENNA | 06-11-1991 | AUGUSTIN |